# EUROPEAN PATENT SPECIFICATION

(11) **EP 1 971 603 B1**
(45) Date of publication and mention of the grant of the patent: **14.03.2012**
(21) Application number: 06849043.2
(22) Date of filing: 11.12.2006
(51) Int. Cl.: C07D 417/04, A61K 31/427, A61P 29/00

(54) **SULFOXIMINE DERIVATIVES AS p38 MAP KINASE INHIBITORS**
SULFOXIMINDERIVATE ASP38-MAP-KINASE-INHIBITOREN
DERIVES DE LA SULFOXIMINE UTILISES COMME INHIBITEURS DE LA P38 MAP KINASE

(30) Priority: 13.12.2005 IN MU15512005
(43) Date of publication of application: 24.09.2008
(73) Proprietor: CADILA HEALTHCARE LIMITED, Amedadabad 380 015 Gujarat (IN)
(72) Inventor: SHETTY, Shankar, Jayram, Gujarat (IN); PATEL, Gautam, D., Gujarat (IN); LOHRAY, Braj, Bhushan, Gujarat (IN); LOHRAY, Vidya, Bhushan, Gujarat (IN); CHAKRABARTI, Ganes, Gujarat (IN); CHATTERJEE, Abhijit, Gujarat (IN); JAIN, Mukul, R., Gujarat (IN); PATEL, Pankaj, Ramanbhai, Gujarat (IN)
(74) Representative: Srinivasan, Ravi Chandran
(86) International application number: PCT/IN2006/000490
(87) International publication number: WO 2007/077574

(56) References cited:
- WO-A-01/74811
- JEFFREY C. BOEHM, JERRY L. ADAMS: EXPERT OPINION ON THERAPEUTIC PATENTS, vol. 10, no. 1, 2000, pages 25-37, XP002439654
- SEIJI MIWATASHI, ET AL.: JOURNAL OF MEDICINAL CHEMISTRY, vol. 48, 2005, pages 5966-5979, XP002439655 cited in the application
- REVESZ L ET AL: "Novel p38 inhibitors with potent oral efficacy in several models of rheumatoid arthritis" BIOORGANIC & MEDICINAL CHEMISTRY LETTERS, OXFORD, GB, vol. 14, 2004, pages 3595-3599, XP003005381 ISSN: 0960-894X
- GUNNAR J. HANSON: EXPERT OPINION ON THERAPEUTIC PATENTS, vol. 7, no. 7, 1997, pages 729-733, XP002439656

## Description

The present invention relates to novel compounds of general formula (I), their stereoisomers, their tautomeric forms, their pharmaceutically acceptable salts and solvates, and pharmaceutical compositions containing them. The present invention also relates to a process of preparing compounds of general formula (I), their stereoisomers their tautomeric forms, their pharmaceutically acceptable salts and solvates, and pharmaceutical compositions containing them.

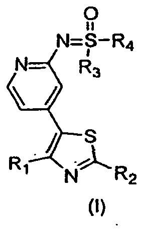

### BACKGROUND AND PRIOR ART

The present invention discloses novel compounds for the treatment of diseases caused by pro-inflammatory cytokines/mediator(s) by inhibiting the p38 MAP kinase.

The etiology and pathogenesis of diseases caused by pro-inflammatory cytokines are not yet fully understood. It is believed that the exposure of a genetically susceptible individual to an environmental factor, possibly an infectious agent, leads to an immune response, which results in the activation of a wide range pro-inflammatory cytokine genes. Cells that play an active role are macrophages, CD4⁺ T-cells, B-cells, dendritic cells and mast cells. They contribute significantly to various aspects of the disease either through cell-cell interactions or through the production of cytokines and other mediators.

The inhibition of cytokine production through transcriptional inhibition is an alternative strategy for therapeutic intervention.

Tumor necrosis factor-α (TNF-α) is a pro-inflammatory cytokine, mainly produced by activated monocytes and macrophages. Excessive production of TNF-α is believed to underlie the progression of many serious inflammatory diseases, such as rheumatoid arthritis (RA), Crohn's disease and psoriasis. Recent clinical data, obtained using chimeric TNF-α antibodies and soluble TNF-α receptor fusion proteins in the treatment of RA, have confirmed the important role of TNF-α in these inflammatory conditions. These agents are generally well tolerated but have drawbacks relating to patient cost, efficiency of production, and administration by injection. Therefore, inflammation research has focused on the development of orally active small molecular inhibitors of cytokine release.

Protein kinases are involved in various cellular responses to extracellular signals. The family of mitogen-activated protein kinases (MAPK) includes Ser/Thr kinases that activate their substrates by dual phosphorylation. MAPKs are reporters of changes in the extracellular milieu, which lead to cellular responses allowing adaptation to changed physiologic and pathologic circumstances. MAPKs function as an "emergency switch" that allows a broad cellular response by turning on the target genes of transcription factors, cytokines, and their surface receptors.

These proteins are therefore considered to be a promising target of future therapeutic compounds that aim to treat diseases caused by pro-inflammatory cytokines/mediator(s).

One particularly interesting MAPK is p38, which is also known as cytokine suppressive anti-inflammatory drug binding protein (CSBP) and RK. Activation of p38 MAP kinase has been observed in cells by a wide variety of stimuli, such as treatment with LPS, UV, anisomycin, or osmotic shock, and by treatment with cytokines, such as IL-1β and TNFα. Inhibition of p38 kinase leads to a blockade in the production of both IL-1β and TNF-α, IL-1β and TNF-α stimulate the production of other proinflammatory cytokines such as IL-6 and IL-8 and have been implicated in acute and chronic inflammatory diseases and in post-menopausal osteoporosis. p38 MAP kinase kinase plays a central role in numerous proinflammatory responses and regulates multiple pathways in inflammation. The p38 MAP kinase is widely expressed in many cell types, including immune, inflammatory and endothelial cells.

The p38 MAP kinase has four isoforms (known till date), namely, p38 MAPKα, p38 MAPKβ, p38 MAPKγ and p38 MAPKδ that are encoded by separate genes. These kinases are all members of the CMGC (CDK (cyclin dependent kinase) MAPK GSK3 (glycogen synthase kinase) CLK (Cdc-2 like kinase)) branch of the human kinome. The p38 MAPKα and p38 MAPKβ kinases are 75% homologous, whereas p38 MAPKγ and p38 MAPKδ are approximately 60% homologous to p38 MAPKα. p38 MAPKα specifically induces the synthesis of proteases such as stromelysin 1 (matrix metalloproteinase 3) or collagenase 1 (matrix metalloproteinase1), which are important for mediating cartilage damage in RA. P38 MAPKβ functions as a survival protein, inducing heat-shock protein 70, a potent antiapoptotic factor induced in the synovial membrane of RA patients. Maintaining cell survival is considered a key feature of p38 MAPKβ activation. Little is known about p38 MAPKγ, which is involved in myocyte differentiation, or about p38 MAPKδ, which acts on microtubule organization (which might be important in the organization of synovial microvessels). The p38 MAPKα isoform has been associated most closely to inflammatory responses. A variety of factors, including stress, endotoxin, cytokines such as TNF-α and IL-1β, and cigarette smoke activate the p38 MAP kinases. Once activated, p38 MAPK phosphorylates downstream substrates to initiate a signal cascade that regulates synthesis of a variety of proinflammatory mediators. TNF-α, IL-1β and COX-2 are among the most important proinflammatory mediators regulated by p38 MAPK. The inhibition of each of these inflammatory mediators has been demonstrated to lead to clinical benefit in diseases caused by pro-inflammatory cytokines/mediator(s), based on approved biologics and NSAIDs. In addition to regulating the production of mediators such as TNF-α and IL-1β, p38 MAPK is activated following the binding of TNF-α, IL-1β and RANKL to their receptors and is responsible for some of their effects. p38 MAPK. inhibition therefore offers two opportunities in intervene in processes involving these cytokines. In addition to inhibiting production of the cytokines themselves, p38 MAPK inhibition has the potential to block deleterious effects of any of the cytokines that may still be produced. For this reason p38 MAPK inhibitors may have the potential for greater efficacy in a variety of diseases than would be predicted by the level of inhibition of cytokine production observed in model systems.

The detailed etiology, physiological function, forms etc, of the p38 MAP kinase and their utility in managing inflammation and other diseases has been described in WO 2006018718 A1, WO 2006094187, WO 2006084017, WO 2006013095, WO 2004024699, WO 2004021988, WO 2004032861 and several other patent and non-patent literature. These are incorporated as reference to the understanding of the mechanism of activation, effects and utility of the p38 MAP kinases.

Several compounds which inhibit the p38 MAP kinases have been developed and are continuing to be developed for the treatment of inflammatory diseases and other indications. These includes those described in WO 2006099495, WO 2006089798, WO 2006067444, WO 20060111416, WO 2006047354, WO 2006040666, WO 2006026235, WO 2006018735, WO 2006018727, WO 2006010082, WO 2005115991, WO 2005108387, WO 2005085244, WO 2005085206, WO 2005085248, WO 2005085249, WO 2004024699, WO 2004014870, WO 2004032861, WO 03068746, WO 03068229, WO 03072569, WO 03032989, WO 03015828, WO 03005999, WO 02007772, WO 02085859, WO 03068746, WO 03068229, WO 9932111, WO 9932110, WO 9932106, WO 9852558, WO 0041698, WO 0043384, WO 9923091, US 20050261354, US 20020065296, US 20030232856, US 20020058678, US 6525046 etc. which are incorporated in their entirety as reference. J. Med. Chem. 2005, 48, 5966-5979 describes 'Novel Inhibitor of p38 MAP Kinase as an Anti-TNF-α Drug: Discovery of N-[4-[2-Ethyl-4-(3-methylphenyl)-1,3-thiazol-5-yl]-2-pyridyl]benzamide (TAK-715) as a Potent and Orally Active Anti-Rheumatoid Arthritis Agent'. US 20040053973 describes substituted 1,3-thiazole compounds, with the following general formula having good p38 MAP kinase activity.

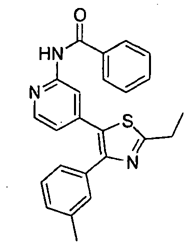

WO2006051826 discloses nitrogenous heterocyclic compound having p38 MAP Kinase activity with the general formula as follows:

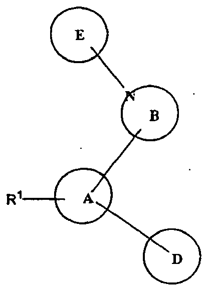

However, since there are no therapies available in the market and looking at the potential of such treatments, there remains a need to develop newer compounds having good activity and safety profile.

We herein disclose novel compounds that demonstrate p38 MAP kinases inhibitory activity and therefore may be suitable for the treatment of Rheumatoid arthritis (RA).

### SUMMARY OF INVENTION

The present invention describes novel compounds useful as inhibitors of p38 MAP kinases. The novel compounds are defined by the general formula (I) below:

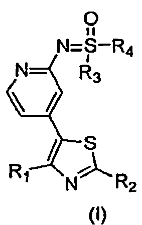

These compounds, or their pharmaceutically acceptable salts, may be, among other things, suitable for the treatment or amelioration of rheumatoid arthritis, pain and its associated pathophysiological conditions wherein p38 plays a significant biological role.

### EMBODIMENTS OF THE INVENTION

In an embodiment of the present invention is provided novel compounds of general formula (I), their stereoisomers, their tautomeric forms, their pharmaceutically acceptable salts, their pharmaceutically acceptable solvates and pharmaceutical compositions containing them or their mixtures and their use in medicine.

In a further embodiment of the present invention is provided a process for the preparation of novel compounds of general formula (I), their stereoisomers,their tautomeric forms, their pharmaceutically acceptable salts and solvates, and pharmaceutical compositions containing them.

In a still further embodiment is provided a pharmaceutical composition which comprises compounds of general formula (I), their tautomeric forms, their stereoisomers, or their pharmaceutically acceptable salts or solvates and a pharmaceutically acceptable carrier, diluent, or excipient.

### DETAILED DESCRIPTION:

The present invention therefore provides compounds of the general formula (I): 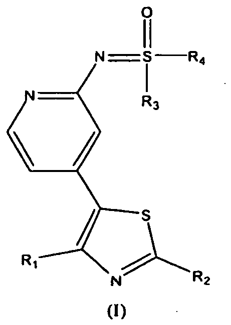 and their stereoisomers, tautomeric forms and their pharmaceutically acceptable salts and solvates, wherein R₁ and R₂ may be the same or different and independently represent hydrogen, optionally substituted groups selected from linear or branched (C₁-C₆)alkyl, linear or branched (C₂-C₆)alkenyl, linear or branched (C₂-C₆)alkynyl, (C₃-C₇)cycloalkyl, (C₃-C₇)cycloalkenyl, aryl, heteroaryl, heterocyclyl groups, each of the cyclic groups may optionally be fused; R₃ and R₄ may be the same or different and may independently be selected from optionally substituted linear or branched (C₁-C₆)alkyl, (C₃-C₇)cycloalkyl, aryl, heteroaryl, heterocyclyl systems, each of these cyclic systems may be optionally fused, or R₃ & R₄ may, together with the sulphur atom to which they are attached, form a 3-7 membered ring system, which may optionally contain from 1-3 heteroatoms selected from N, O or S.

The aryl group may be an aromatic system containing one, two or three rings wherein such rings may be attached together in a pendant manner or may be fused; in a preferred embodiment such aryl groups are selected from phenyl, naphthyl, tetrahydronaphthyl, indane, biphenyl groups;

The heteroaryl group represents 5 to 8 membered aromatic radicals, which may be single or fused containing one or more hetero atoms selected from O, N or S; in a preferred embodiment such groups may be selected from pyridyl, thienyl, furyl, pyrrolyl, oxazolyl, thiazolyl, isothiazolyl, imidazolyl, isoxazolyl, oxadiazolyl, thiadiazolyl, triazolyl, tetrazolyl, benzopyranyl, benzopyranonyl, benzofuranyl, benzothienyl, indolinyl, indolyl, azaindolyl, azaindolinyl, benzodihydrofuranyl, benzodihydrothienyl, pyrazolopyrimidinyl, pyrazolopyrimidonyl, azaquinazolinyl, azaquinazolinoyl, pyridofuranyl, pyridothienyl, thienopyrimidyl, thienopyrimidonyl, quinolinyl, pyrimidinyl, pyrazolyl, quinazolinyl, quinazolonyl, pyrimidonyl, pyridazinyl, triazinyl, benzoxazinyl, benzoxazinonyl, benzothiazinyl, benzothiazinonyl, benzoxazolyl, benzothiazolyl, benzimidazolyl, benzotriazolyl, phthalazynil, naphthylidinyl, purinyl carbazolyl, phenothiazinyl, phenoxazinyl groups;

The term "heterocyclyl" represents saturated, partially saturated and unsaturated ring-shaped radicals, the heteroatoms selected from nitrogen, sulfur and oxygen; in a preferred embodiment the heterocyclyl is selected from aziridinyl, azetidinyl, pyrrolidinyl, imidazolidinyl, piperidinyl, piperazinyl, 2-oxopiperidinyl, 4-oxopiperidinyl, 2-oxopiperazinyl, 3-oxopiperazinyl, morpholinyl, thiomorpholinyl, 2-oxomorpholinyl, azepinyl, diazepinyl, oxapinyl, thiazepinyl, oxazolidinyl, thiazolidinyl, and the like; examples of partially saturated heterocyclic radicals include dihydrothiophene, dihydropyran, dihydrofuran, dihydrothiazole groups.

The substituents on any of the groups described above may be selected from hydroxyl, oxo, halo, thio, nitro, amino, imino, cyano, formyl, or optionally substituted groups selected from alkyl, haloalkyl, hydroxyalkyl, aminoalkyl, thioalkyl, alkoxy, haloalkoxy, alkoxyalkyl, acyl, monosubstituted or disubstituted amino, carboxylic acid and its derivatives such as esters and amides.

When any of the groups are further substituted, the substituents may be selected from any of the groups described above, alone or in combination with other suitable groups mentioned in the specification.

In a further preferred embodiment the groups, radicals described above may be selected from:
- the "alkyl" group used either alone or in combination with other radicals, denotes a linear or branched radical containing one to eight carbons, selected from methyl, ethyl, *n*-propyl, *iso*-propyl, *n*-butyl, *sec*-butyl, *tert*-butyl, amyl, *t*-amyl, *n*-pentyl, *n*- hexyl, *iso*hexyl, heptyl, octyl and the like;
- the "alkenyl" group used either alone or in combination with other radicals, is selected from a radical containing from two to twelve carbons, more preferably groups selected from vinyl, allyl, 2-butenyl, 3-butenyl, 2-pentenyl, 3-pentenyl, 4- pentenyl, 2-hexenyl, 3-hexenyl, 4-hexenyl, 5-hexenyl, 2-heptenyl, 3-heptenyl, 4-heptenyl, 5-heptenyl, 6-heptenyl and the like; the "alkenyl" group includes dienes and trienes of straight and branched chains;
- the "alkynyl" group used either alone or in combination with other radicals, is selected from a linear or branched radical containing two to twelve carbon atoms, more preferably thynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 3-hexynyl, 4-hexynyl, 5-hexynyl, and the like. The term "alkynyl" includes di- and tri-ynes;
- the "cycloalkyl", or "alicyclic" group used either alone or in combination with other radicals, is selected from a cyclic radical containing three to seven carbons, more preferably cyclopropyl, cyclobutyl, cyclopentyl, cyclohexyl, cycloheptyl and the like; The terms "bicycloalkyl" means more than one cycloalkyl groups fused together;
- the "cycloalkenyl" group used either alone or in combination with other radicals, are preferably selected from cyclopropenyl, 1-cyclobutenyl, 2-cylobutenyl, 1-cyclopentenyl, 2-cyclopentenyl, 3-cyclopentenyl, 1-cyclohexenyl, 2-cyclohexenyl, 3-cyclohexenyl, 1-cycloheptenyl, cycloheptadienyl, cycloheptatrienyl, and the like;
- the "alkoxy" group used either alone or in combination with other radicals, is selected from groups containing an alkyl radical, as defined above, attached directly to an oxygen atom, more preferably groups selected from methoxy, ethoxy, n-propoxy, iso-propoxy, n-butoxy, t-butoxy, iso-butoxy, pentyloxy, hexyloxy, and the like;
- the "alkenoxy" group used either alone or in combination with other radicals, is selected from groups containing an alkenyl radical, as defined above, attached to an oxygen atom, more preferably selsected from vinyloxy, allyloxy, butenoxy, pentenoxy, hexenoxy, and the like;
- the "haloalkyl" group is selected from an alkyl radical, as defined above, suitably substituted with one or more halogens; such as perhaloalkyl, more preferably, perfluoro(C₁-C₆)alkyl such as fluoromethyl, difluoromethyl, trifluoromethyl, fluoroethyl, difluoroethyl, trifluoroethyl, mono or polyhalo substituted methyl, ethyl, propyl, butyl, pentyl or hexyl groups;
- the "haloalkoxy" group is selected from suitable haloalkyl, as defined above, directly attached to an oxygen atom, more preferably groups selected from fluoromethoxy, chloromethoxy, fluoroethoxy chloroethoxy and the like;
- the "acyl" group used either alone or in combination with other radicals, is selected from a radical containing one to eight carbons, more preferably selected from formyl, acetyl, propanoyl, butanoyl, iso-butanoyl, pentanoyl, hexanoyl, heptanoyl, benzoyl and the like, which may be substituted;
- the "mono-substituted amino" group used either alone or in combination with other radicals, represents an amino group substituted with one group selected from (C₁-C₆)alkyl, substituted alkyl, aryl, substituted aryl or arylalkyl groups as defined earlier, more preferably such groups are selected from methylamine, ethylamine, *n-*propylamine, *n*-butylamine, *n*-pentylamine and the like;
- the 'disubstituted amino" group used either alone or in combination with other radicals, represents an amino group, substituted with two radicals that may be same or different selected from (C₁-C₆)alkyl, substituted alkyl, aryl, substituted aryl, or arylalkyl groups, as defined above, more preferably the groups are selected from dimethylamino, methylethylamino, diethylamino, phenylmethyl amino and the like;
- the "arylamino" used either alone or in combination with other radicals, represents an aryl group, as defined above, linked through amino having a free valence bond from the nitrogen atom, more preferably the groups are selected from phenylamino, naphthylamino, N-methyl anilino and the like;
- the "carboxylic acid" group, used alone or in combination with other radicals, denotes a -COOH group, and includes derivatives of carboxylic acid such as esters and amides;
- the "ester" group used alone or in combination with other radicals, denotes -COO-group, and includes carboxylic acid derivatives, more preferably the ester moieties are selected from alkoxycarbonyl, such as methoxycarbonyl, ethoxycarbonyl, and the like, which may optionally be substituted; aryloxycarbonyl group such as phenoxycarbonyl, napthyloxycarbonyl, and the like, which may optionally be substituted;
- the "hydroxyalkyl" group used either alone or in combination with other radicals, is selected from an alkyl group, as defined above, substituted with one or more hydroxy radicals, more preferably the groups are selected from hydroxymethyl, hydroxyethyl, hydroxypropyl, hydroxybutyl, hydroxypentyl, hydroxyhexyl and the like;
- the "thioalkyl" group used either alone or in combination with other radicals, denotes an alkyl group, as defined above, attached to a group of formula -SR', where R' represents hydrogen, alkyl or aryl group, e.g. thiomethyl, methylthiomethyl, phenylthiomethyl and the like, which may be optionally substituted.

Suitable groups and substituents on the groups may be selected from those described anywhere in the specification.

In a further preferred embodiment, the compounds of the present invention may be selected from
S-Cyclopentyl-S-phenyl-N-[4-(2-ethyl-4-(3-methylphenyl)-1,3-thiazol-5-yl)-pyridine-2-yl]-sulfoximine;
S,S-Dicyclohexyl-N-[4-(2-ethyl-4-m-tolyl-thiazol-5-yl)-pyridine-2-yl]-sulfoximine; S-methyl-S-4-methoxyphexyl-N-[4-(2-ethyl-4-m-tolyl-thiazol-5-yl)-pyridine-2-yl]-sulfoximine;
S-Methyl-S-phenyl-N-[4-(2-ethyl-4-(3-methylphenyl)-1,3-thiazol-5-yl)-pyridine-2-yl]-sulfoximine;
(+)-(S)-yethyl-S-phenyl-N-[4-(2-ethyl-4-(3-methylphenyl)-1,3-thiazol-5-yl)-pyridine-2-yl]-sulfoximine;
(-)-(S)-Methyl-S-phenyl-N-[4-(2-ethyl-4-(3-methylphenyl)-1,3-thiazol-5-yl)-pyridine-2-yl]-sulfoximine;
S-Methyl-S-(4-fluorophenyl)-N-[4-(2-ethyl-4-(3-methylphenyl)-1,3-thiazol-5-yl)-pyridine-2-yl]-sulfoximine;
S-Isobutyl-S-phenyl-N-[4-(2-ethyl-4-m-tolyl-thiazol-5-yl)-pyridin-2-yl]-sulfoximine;
S-(3-Fluorophenyl)-S-methyl-N-[4-(2-ethyl-4-m-tolyl-thiazol-5-yl)-pyridin-2-yl]-sulfoximine;
S-(3-Methylphenyl)-S-methyl-N-[4-(2-ethyl-4-m-tolyl-thiazol-5-yl)-pyridin-2-yl]-sulfoximine;
S, S-Diphenyl-N-[4-(2-ethyl-4-m-tolyl-thiazol-5-yl)-pyridin-2-yl]-sulfoximine;
S-(2-Methylphenyl)-S-methyl-N-(4-(2-ethyl-4-m-tolyl-thiazol-5-yl)-pyridin-2-yl]-sulfoximine;
S-Isopropyl-S-phenyl-N-[4-(2-ethyl-4-(3-methylphenl)-1,3-thiazol-5-yl)-pyridine-2-yl]-sulfoximine;
S-Ethyl-S-phenyl-N-[4-(2-ethyl-4-(3-methylphenyl)-1,3-thiazol-5-yl)-pyridine-2-yl]-sulfoximine;
S-Methyl-S-4-methylphenyl-N-[4-(2-ethyl-4-(3-methylphenyl)-1,3-thiazol-5-yl)-pyridine-2-yl]-sulfoximine;
Di-n-butyl-N-[4-(2-ethyl-4-(3-methylplienyl)-1,3-thiazol-5-yl)-pyridine-2-yl]-sulfoximine;
S-Methyl-S-(3-chloro-4-flurophenyl)-N-[4-(2-ethyl-4-(3-methylphenyl)-1,3-thiazol-5-yl)-pyridine-2-yl]-sulfoximine;
1-Imino-N-[4-(2-ethyl-4-(3-methylphenyl)-1,3-thiazol-5-yl)-pyridihe-2-yl]-tetrahydro-2H-thiopyran-1-oxide;
1-Imino-N-[4-(2-ethyl-4-(3-methylphenyl)-1,3-thiazol-5-yl)-pyridine-2-yl]-tetrahydrothiophene-1-oxide;
(-)-(S)-Methyl-S-phenyl-N-[4-(2-ethyl-4phenyl)-1,3-thiazole-5-yl)-pyridine-2-yl]-sulfoximine;
(+)-S-Methyl-S-phenyl-N-[4-(2-ethyl-4phenyl)-1,3-thiazole-5-yl)-pyridine-2-yl]-sulfoximine;
S-Isopropyl-S-phenyl-N-[4-(2-ethyl-4-phenyl)-1,3-thiazole-5-yl)-pyridine-2-yl]-sulfoximine;
S-(4-Methoxyphenyl)-S-methyl-N-[4-(2-ethyl-4-phenyl)-1,3-thiazole-5-yl)-pyridine-2-yl]-sulfoximine;
S-Methyl-S-(3-methylphenyl)-N-[4-{2-ethyl-4-phenyl-thiazol-5-yl}-pyridine]-sulfoximine;
S-Methyl-S-(3-fluorophenyl)-N-[4-{2-ethyl-4-phenyl-thiazol-5-yl}-pyridine]-sulfoximine;
4-[2-Ethyl-4-phenyl-thiazol-5-yl]-2-(dicycloxyl sulfoximine)-pyridine;
S-Methyl-S-(4-fluorophenyl-N-[4-{2-ethyl-4-phenyl-thiazol-5-yl}-pyridine]-sulfoximine;
S-Cyclopentyl-S-phenyl-N-[4-{2-ethyl-4-phenyl-thiazol-5-yl}-pyridine]-sulfoximine;
(-)-S-Cyclopentyl-S-phenyl-N-[4-(2-ethyl-4-phenyl)-1,3-thiazol-5-yl)-pyridine-2-yl]-sulfoximine;
(+)-S-Cyclopentyl-S-phenyl-N-[4-(2-ethyl-4-phenyl)-1,3-thiazol-5-yl)-pyridine-2-yl]-sulfoximine;
S-Methyl-S-phenyl-N-[4-(2-ethyl-4-phenyl)-1,3-thiazol-5-yl)-pyridine-2-yl]-sulfoximine;
S,S-Diphenyl-N-[4-(2-ethyl-4-phenyl-thiazol-5-yl)-pyridin-2-yl]-sulfoximine;
(-)-S-Methyl-S-phenyl-N-{4-[2-ethyl-4-(4-fluoro-phenyl)-thiazol-5-yl]-pyridin-2-yl}-sulfoximine;
S-(4-Methoxy-phenyl)-S-methyl-N-{4-[2-ethyl-4-(4-fluoro-phenyl)-thiazol-5-yl]-pyridin-2-yl}-sulfoximine;
S-Isopropyl-S-phenyl-N-{4-[2-ethyl-4-(4-fluoro-phenyl)-thiazol-5-yl]-pyridin-2-yl}-sulfoximine;
(-)-Isopropyl-S-phenyl-N-{4-[2-ethyl-4-(4-fluoro-phenyl)-thiazol-5-yl]-pyridin-2-yl}-sulfoximine;
(+)-S-isopropyl-S-phenyl-N-{4-[2-ethyl-4-(4-fluoro-phenyl)-thiazol-5-yl]-pyridin-2-yl}-sulfoximine;
S-Ethyl-S-phenyl-N-{4-[2-ethyl-4-(4-fluoro-phenyl)-thiazol-5-yl]-pyridin-2-yl}-sulfoximine;
S-Methyl-S-phenyl-N-[4-(2-ethyl-4-(4-fluoro-phenyl)-1,3-thiazol-5-yl)-piridine-2-yl]-sulfoximine;
(+)-S-Methyl-S-phenyl-N-[4-(2-ethyl-4-(4-fluoro-phenyl)-1,3-thiazol-3-yl)-pyridine-2-yl]-sulfoximine;
S-Methyl-S-4-fluoro-phenyl-N-[4-(2-ethyl-4-(4-fluoro-phenyl)-1,3-thiazol-5-yl)-pyridine-2-yl]-sulfoximine;
S-Cyclopentyl-S-phenyl-N-[4-(2-ethyl-4-(4-fluorophenyl)-1,3-thiazol-5-yl)-pyridine-2-yl]-sulfoximine;
(-)-S-Methyl-S-4-fluoro-phenyl-N-[4-(2-ethyl-4-(4-fluoro-phenyl)-1,3-thiazol-5-yl)-pyridine-2-yl]-sulfoximine;
(+)-S-Methyl-S-4-fluoro-phenyl-N-[4-(2-ethyl-4-(4-fluoro-phenyl)-1,3-thiazol-5-yl)-pyridine-2-yl]-sulfoximine;
S,S-Diphenyl-N-[4-(2-ethyl-4-(4-fluoro phenyl)-thiazol-5-yl)-pyridin-2-yl]-sulfoximine;
S-Methyl-S-3-methylphenyl-N-[4-(2-ethyl-4-fluorophenyl-thiazol-S-yl)-pyridine]-sulfoximine;
S-Methyl-S-3-fluorophenyl-N-[4-{2-ethyl-4-(4-fluorophenyl)-thiazol-5-yl}-pyridine-2yl]-sulfoximine;
S-Cyclohexyl-S-phenyl-N-{4-[2-ethyl-4-(4-fluorophenyl)-thiazol-5-yl)-pyridine}-sulfoximine;
S-Methyl-S-phenyl-N-[4-(2-ethyl-4-m-benzoic acid-thiazol-5-yl)-pyridine-2-yl]-sulfoximine;
S-Methyl-S-phenyl-N-[4-(2-ethyl-4-(3-fluoro-plienyl)-1,3-thiazol-5-yl)-pyridine-2-yl]-sulfoximine;
S-Isopropyl-S-phenyl-N-[4-(2-ethyl-4-(3-fluoro-phenyl)-1,3-thiazol-5-yl)-pyridine-2-yl]-sulfoximine;
S-Cyclopentyl-S-phenyl-N-[4-(2-ethyl-4-(3-fluoro-phenyl)-1,3-thiazol-5-yl)-pyridine-2-yl]-sulfoximine;
S-Methyl-S-phenyl-N-{4-[2-ethyl-4-naphthalene-1-yl-thiazol-5-yl]-pyridin-2-yl}-sulfoximine;
S-Methyl-S-phenyl-N-{4-[2-ethyl-4-(3-trifluoromethylphenyl)-thiazol-5-yl]-pyridin-2-yl}-sulfoximine;
S-Methyl-S-phenyl-N-{4-[2-ethyl-4-(4-methoxyphenyl)-thiazol-5-yl]-pyridin-2-yl}-sulfoximine;
S-Methyl-S-phenyl-N-{4-[2-(4-methylsulfanyl-phenyl)-4-m-tolyl-thiazol-5-yl]-pyridin-2-yl}-sulfoximine;
S-Methyl-S-phenyl-N-{4-[2-(4-methylsulfinyl-phenyl)-4-m-tolyl-thiazol-5-yl]-pyridin-2-yl}-sulfoximine;
S-Methyl-S-phenyl-N-{4-[2-ethyl-4-(3,4-difluorophenyl)-thiazol-5-yl]-pyridin-2-yl}-sulfoximine;
S-Methyl-(4-fluorophenyl)-N-{4-[2-ethyl-4-(3,4-difluorophenyl)-thiazol-5-yl]-pyridin-2-yl}-sulfoximine;
S-isopropyl-S-phenyl-N-{4-[2-ethyl-4-(3,4-difluorophenyl)-thiazol-5-yl]-pyridin-2-yl}-sulfoximine;
S-isopropyl-S-phenyl-N-{4-(2-ethyl-4-(3,5-difluorophenyl)-thiazol-5-yl]-pyridin-2-yl}-sulfoximine;
S-Methyl-S-phenyl-N-{4-[2-ethyl-4-(3,5-difluorophenyl)-thiazol-5-yl]-pyridin-2-yl}-sulfoximine;
S,S-Diphenyl-N-{4-[2-ethyl-4-(3,5-difluorophenyl)-thiazol-5-yl]-pyridin-2-yl}-sulfoximine;
S-Methyl-S-phenyl-N-{4-[2-ethyl-4-p-tolyl-thiazol-5-yl]-pyridin-2-yl}-sulfoximine; S-Cyclopentyl-S-phenyl-N-{4-[2-(2,6-difluorophenyl)-4-(3-fluorophenyl)-(1,3)-thiazol-5-yl]-pyridin-2-yl}-sulfoximine;
S-Isopropyl-S-phenyl-N-{4-[2-(2,6-difluorophenyl)-4-(3-fluorophenyl)-(1,3)-thiazol-5-yl]-pyridin-2-yl}-sulfoximine;
S-Cyclohexyl-S-phenyl-N-{4-[2-(2,6-difluorophenyl)-4-(3-fluorophenyl)-(1,3)-thiazol-5-yl]-pyridin-2-yl}-sulfoximine;
S-Cyclohexyl-S-phenyl-N-{4-[2-(4-fluorophenyl)-4-(3-fluorophenyl)-(1,3)-thiazol-5-yl]-pyridin-2-yl}-sulfoximine;
S-Isopropyl-S-phenyl-N-{4-[2-(4-fluorophenyl)-4-(3-fluorophenyl)-(1,3)-thiazol-5-yl]-pyridin-2-yl}-sulfoximine;
Methane sulfonate salt of (+)-S-Isopropyl-S-phenyl-N-{4-[2-ethyl-4-(4-fluorophenyl)-(1,3)-thiazol-5-yl]-pyridin-2-yl}-sulfoximine;
N-Oxide of (+)-S-isopropyl,S-phenyl-N-{4-[2-ethyl-4-(4-fluorophenyl)-(1,3)-thiazol-5-yl]-pyridin-2-yl}-sulfoximine methane sulfonate salt;
S-Cyclopentyl-S-phenyl-N-[4-(3-fluorophenyl)-1,3-thiazol-5-yl)-pyridine-2-yl]-sulfoximine;
S-Methyl-S-phenyl-N-[4-(3-fluorophenyl)-1,3-thiazol-5-yl)-pyridine-2-yl]-sulfoximine;
S-Isopropyl-S-phenyl-N-[4-(3-fluorophenyl)-1,3-thiazol-5-yl)-pyridine-2-yl]-sulfoximine;
S-Methy)-S-phenyl-N-[4-(4-fluoropbenyl)-1,3-thiazol-5-yl-pyridine-2-yl]-sulfoximine;
S-Isopropyl-S-phenyl-N-[4-(4-fluorophenyl)-1,3-thiazol-5-yl)-pyridine-2-yl]-sulfoximine;
S-Cyclopentyl-S-phenyl-N-[4-(4-fluorophenyl)-1,3-thiazol-5-yl)-pyridine-2-yl]-sulfoximine;
S-Methyl-S-phenyl-N-[4-phenyl-(1,3)-thiazol-5-yl-yridine-2-yl]-sulfoximine;
S-Isopropyl-S-phenyl-N-[4-phenyl-1,3-thiazol-5-yl)-pyridine-2-yl]-sulfoximine; S-Cyclopentyl-S-phenyl-N-[(4-phenyl-thiazol-5-yl)-pyridine-2-yl]- sulfoximine.

The compounds of formula (I), where all symbols are as defined earlier, may be synthesized using the methods described below, or in combination with suitable modifications of conventional techniques known to those skilled in the art of organic synthesis, or variations thereof as appreciated by those skilled in the art. Preferred methods include, but not limited to those described below:

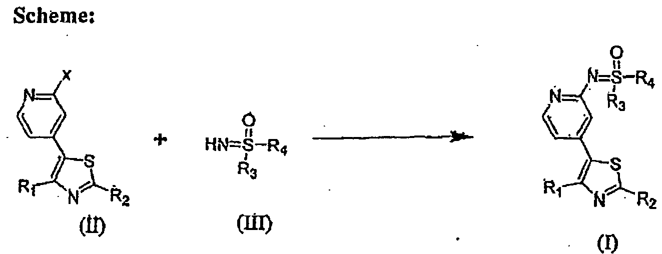

Reacting a compound of formula (II) wherein 'X' represents suitable leaving group such as iodo, bromo, and the like as are known in the literature and all other symbols are as defined earlier, with sulfoximine compound of formula (III) wherein all the symbols are as defined earlier using suitable coupling catalyst(s) such as different palladium-catalysts like palladium acetate, copper salts, such as copper(I) iodide, in the presence of suitable ligand(s) like N,N'-dimethylethyl diamine (DMEDA), and in the presence of suitable inorganic base(s) such as cesium carbonate, cesium acetate, potassium carbonate, potassium phosphate, potassium hydroxide, sodium hydroxide, sodium carbonate, lithium hydroxide, sodium hydride and potassium hydride to yield a compound of formula (I). The reaction may be carried out in suitable solvents selected from toluene, DMSO, dioxane and the like or mixture(s) thereof and the reaction may be carried out at a temperatures in the range of 0 °C to reflux temperature of the solvent(s) used and the reaction time may range from 1 to 72 hours.

It will be appreciated that in any of the above mentioned reactions any reactive group in the substrate molecule may be protected, according to conventional chemical practice. Suitable protecting groups in any of the above mentioned reactions are those used conventionally in the art.

The invention is explained in greater detail by the examples given below, which are provided by way of illustration only and therefore should not be construed to limit the scope of the invention.
*1H NMR spectral data given at the end of each of the compounds (vide infra) are recorded using a 300 MHz spectrometer (Bruker AVANCE-300) and reported in δ scale. Until and otherwise mentioned the solvent used for NMR is CDCl₃ using tetramethyl silane as the internal standard.*

### EXAMPLE 1

### S-Cyclopentyl-S-phenyl-N-[4-(2-ethyt-4-(3-methylphehyl)-1,3-thiazol-5-yl)-pyridine-2-yl]-sulfoximine

To a stirred solution of 4-[2-ethyl-4-(3-methyl-phenyl)-1,3-thizol-5-yl]-2-iodo pyridine (0.15g) in dry toluene was added S-Cyclopentyl-S-phenyl sulfoximine (77.27 mg), copper (I) iodide (7 mg), N, N'-Dimethyl-ethylene diamine (6.5 mg) and Cesium carbonate (0.3 g). The reaction mixture was heated to elevated temperature (at least 100 °C) under nitrogen atmosphere overnight. After completion of reaction, the contents were poured into water and extracted with ethyl acetate. The organic layer was dried over Na₂SO₄ and solvents were evaporated under vacuum to give brown oil. The crude product was flash chromatographed over silica. Elution with 50 % ethyl acetate in hexane afforded desired product as a white solid (0.093 mg). Yield =32%
¹H NMR [CDCl₃, 300 MHz]: 1.40-1.45(3H, t, J=7.57Hz); 1.68-1.75(4H, broad); 2.01 - 2.17(3H, broad); 2.3(1H, s); 2.34-2.36(1H, broad); 3.75-3.8(1H, m); 6.54-6.51 (1H, dd., 1.56Hz, J=5.29Hz); 6.87(1H, s); 7.1-7.17(3H, m); 7.36(1H, s); 7.50-7.58 (3H, m); 7.86-7_{:}91 (3H, m)

### EXAMPLE 2

### S,S-dicydohexyl-N-[4-(2-ethyl-4-m-tolyl-thiazol-5-yl)-pyridine-2-yl]-sulfoximine

To a stirred solution of 4-[2-ethyl-4-(3-methyl-phenyl)-1,3-thiazol-5-yl]-2-iodo pyridine (0.15g) in dry toluene was added S,S-Dicyclohexyl sulfoximine (77.27 mg), copper (I) iodide (7 mg), N, N'-Dimethyl-ethylene diamine (6.5mg) and Cesium carbonate (0.3 g). The reaction mixture was heated to elevated temperatures (at least 100 °C) under nitrogen atmosphere overnight. After completion, the contents were poured over water and extracted with ethyl acetate. The organic layer was dried over Na₂SO₄ and solvents were evaporated under table vacuum, to give brown oil. The crude product was flash chromatographed over silica. Elution with ethyl, acetate in hexane afforded desired product (0.09 mg) as white solid. (yield=32 %).
¹H NMR (CDCl₃, 300Hz): 1.2(9H,m); 1.4(3H, t, J =7.5Hz); 1.6(4H, m); 1.8(4H, m); 2.1(4H,m); 2.3(3Hm); 3.05(2H,q, J =7.59Hz); 3.4(2H,m); 6.5(1H,dd,J=1.47), 6,925(1H,s); 7.1(2H,m);7.4(1H,s), 8.02(1H,dd,J=5.32Hz)

The following compounds were prepared by procedures similar to those described in examples 1 or 2 with appropriate variations of reactants, reaction conditions and quantities of reagents, as can be appreciated by a person skilled in the art.

### EXAMPLE 3

### S-Methyl-S-4-methoxyphenyl-N-[4-(2-ethyl-4-m-tolyl-thiazol-5-yl)-pyridine-2-yl]-sulfoximine

¹H NMR (CDCl₃, 300Hz) 1.43 (3H, t, J = 7.5Hz); 2.32 (3H, s); 3.06 (2H, q, J = 7.56 Hz); 3.35 (3H,s); 3.87(3H,s); 6.61(1H,m); 6.88(1H,s); 7.09(2H,dd); 7.26 (3H,m); 7.38 (1H,s); 7.92 (3H,m) Yield =33 %

### EXAMPLE 4

### S-Methyl-S-phenyl-N-[4-(2-ethyl-4-(3-methylphenyl)-1,3-thiazol-5-yl)-pyridine-2-yl]-sulfoximine

¹H NMR (CDCl₃, 300M Hz CDCl₃, 300 MHz) 1.41=1.46(3H, t, J=7.57 Hz), 3.03-3.10(2H, t, J=7.55 Hz), 3.36(3H, s), 6.5-9-6.61(1H, d, J=4.56 Hz), 6.89(1H, s), 7.09-7.21(3H, m), 7.38(1H, s), 7.53-7.65(3H, m), 7.92-7.94(1H, d, J=5.13 Hz), 8.00-8.03(1H, d, J=7.17 Hz) Yield =39 %.

### EXAMPLE 5

### (+)-(S)-methyl-S-phenyl-N-[4-(2-ethyl-4-(3-methylphenyl)-1,3-thiazol-5-yl)-pyridine-2-yl]-sulfoximine

¹H NMR (CDCl₃, 300M Hz) 1.41 (t, 3H, J=7.59 Hz), 2.32 (s, 3H), 3.03 (q, 2H, J=7.59 Hz), 3.37 (s, 3H), 6.59 (dd, 1H, J=1.56 & 6.9 Hz), 6.89 (s, 1H), 7.11 (m, 3H), 7.39 (s, 1H), 7.56 (m, 3H), 7.92 (d, 1H, J=5.3 Hz), 8.0 (dd, 2H, J=1.59 & 8.46Hz). Yield =56 %.

### EXAMPLE 6

### (-)-S-methyl-S-phenyl-N-[4-(2-ethyl-4-(3-methylphenyl)-1,3-thiazol-5-yl)-pyridine-2-yl]-sulfoximine

¹H NMR (CDCl₃, 300M Hz) 1.41 (t, 3H, J=7.59 Hz), 2.32 (s, 3H), 3.03 (q, 2H, J=7.59 Hz), 3.37 (s, 3H), 6.59 (dd, 1H, J=1.56 & 6.9 Hz), 6.89 (s, 1H), 7.11 (m, 3H), 7.39 (s, 1H), 7.56 (m, 3H), 7.92 (d, 1H, J=5.3 Hz), 8.0 (dd, 2H, J=1.59 & 8.46Hz). Yield = 45 %.

### EXAMPLE 7

### S-Methyl-S-(4-fluorophenyl)-N-[4-(2-ethyl-4-(3-methylphenyl)-1,3-thiazol-5-yl)-pyridine-2-yl]-sulfoximine

¹H NMR (CDCl₃, 300 MHz) 1.41-1.46(3H, t, J=7.59 Hz), 2.32(3H, s), 3.03-3.11(2H, t, J=7.59 Hz), 3.36(3H, s), 6.61-6.63(1H, dd, J=1.56 & 5.31 Hz), 6.88(1H, s), 7.12-7.23(5H, m), 7.38(1H, s), 7.92-7.94(2H, d, J=5.37 Hz), 7.99-8.04(2H, dd, J=1.8 & 5.1 Hz) Yield =64 %.

### EXAMPLE 8

### S-Isobutyl-S-phenyl-N-[4-(2-ethyl-4-m-tolyl-thiazol-5-yl)-pyridin-2-yl]-sulfoximine

¹NMR (CDCl₃, 300 MHz) 0.95 (d, 3H, J=6.6 Hz), 1.04 (d, 3H, J=6.6Hz), 1.41 (t, 3H, J=7.5 Hz), 2.31 (s, 3H), 3.03 (q, 2H, J=7.5 Hz), 3.23 (dd, 1H, J=6.6 & 6.6 Hz), 3.41 (dd, 2H, J=5.7 & 6 Hz), 6.55 (dd, 1H, J=1.5 & 5.4 Hz), 6.87 (s, 1H), 7.11 (m, 3H), 7.37 (s, 1H), 7.51 (m, 3H), 7.90 (d, 1H, J=5.4 Hz), 7.95 (m, 2H). Yield =39 %.

### EXAMPLE 9

### S-(3-Fluorophenyl)-S-methyt-N-[4-(2-ethyl-4-m-tolyl-thiazol-5-yl)-pyridin-2-yl]-sulfoximine

¹H NMR (CDCl₃, 300 MHz) 1.41 (t, 3H, J=7.5 Hz), 2.32 (s, 3H), 3.03 (q, 2H, J=7.5 Hz), 3.36 (s, 3H), 6.61 (dd, 1H, J=1.5 & 5.3 Hz), 6.88(s, 1H), 7.12 (m, 3H), 7.31 (m, 1H), 7.39 (s, 1H), 7.54 (m, 1H), 7.70 (d, 1H, J=7.9 Hz), 7.79 (d, 1H, J=7.9 Hz), 7.91 (d, 1H, J=5.3 Hz). Yield =93 %.

### EXAMPLE 10

### S-(3-Methylphenyl)-S-methyl-N-[4-(2-ethyl-4-m-tolyl-thiazol-5-yl)-pyridin-2-yl]-sulfoximine

¹H NMR (CDCl₃, 300 MHz) 1.41 (t, 3H, J=7.5 Hz), 2.31 (s, 3H), 2.45 (s, 3H), 3.03 (q, 2H, J=7.5 Hz), 3.36 (s, 3H), 6.59 (dd, 1H, J=1.5 & 5.3 Hz), 6.89(s, 1H), 7.11 (m, 3H), 7.39 (m, 3H), 7.77 (m, 1H), 7.85 (s, 1H), 7.94 (d, 1H, J=5.3 Hz). Yield =45 %

### EXAMPLE 11

### S, S-Diphenyl-N-[4-(2-ethyl-4-m-tolyl-thiazol-5-yl)-pyridin-2-yl]-sulfoximine

¹H NMR (CDCl₃, 300 MHz) 1.23 (t, 3H, J=7.3 Hz), 2.31 (s, 3H), 3.04 (q, 2H, J=7.5 Hz), 6.58 (dd, 1H, J=1.5 & 5.3 Hz), 7.05 (s, 1H), 7.11 (m, 3H), 7.38 (s, 1H), 7.47 (m, 6H), 7.89 (d, 1H, J=5.1 Hz), 8.03 (dd, 4H, J=1.3 & 7.7 Hz). Yield =19 %.

### EXAMPLE 12

### S-(2-Methylphenyl)-S-methyl-N-[4-(2-ethyl-4-m-tolyl-thiazol-5-yl)-pyridin-2-yl]-sulfoximine

¹H NMR (CDCl₃, 300 MHz) 1.40 (t, 3H, J=7.5 Hz), 2.31 (s, 3H), 2.615 (s, 3H), 3.02 (q, 2H, J=7.5 Hz), 3.35 (s, 3H), 6.6 (dd, 1H, J=1.4 & 5.3 Hz), 6.8 (s, 1H), 7.11 (m, 3H), 7.27 (m, 1H), 7.37 (M, 2H), 7.42 (m, 1H), 7.90 (d, 1H, J=5.3 Hz), 8.16 (d, 1H, J=7.86 Hz). Yield =59 %.

### EXAMPLE 13

### S-Isopropyl-S-phenyl-N-[4-(2-ethyl-4-(3-methylphenyl)-1,3-thiazol-5-yl)-pyridine-2-yl]-sulfoximine

¹H NMR (CDCl₃, 300 MHz) 1.27-1.29( 3H, m ); 1.41-1.46( 3H, t, J=7.53Hz ); 2.30( 3H, s) 3.02-3.1( 2H, q, J=7.57Hz ); 3.57-3.67( 1H, m ); 6.54-6.56( 1H, dd, J=1.53Hz, J=5.32 Hz ); 6.89( 1H, s ); 7.1-7.2( 3H, m ); 7.37( 1H, s ); 7.5-7.6( 3H, m ); 7.87-7.91( 3 H, m) Yield =65 %

### EXAMPLE 14

### S-Ethyl-S-Phenyl-N-[4-(2-ethyl-4-(3-methylphenyl)-1,3-thiazol-5-yl)-pyridine-2-yl]-sulfoximine

¹H NMR[CDCl₃ 300 MHz] 1.24-1.29 (3H, t, J=7.34Hz ); 1.41-1.46( 3H, t, J=7.56Hz ); 2.31 ( 3H, s ); 3.03-3.1 ( 2H, q, J=7.56Hz ); 3.46-3.53( 2H, q, J=7.39Hz ); 6.57-6.59( 1H,dd, J=1.45Hz, J=5.33Hz ); 6.9( 1H, s ); 7.11-7.19( 3H, m ); 7.38( 1H, s ); 7.52-7.62
(3H, m); 7.91=7.96(3H, m). Yield =57 %.

### EXAMPLE 15

### S-Methyl-S-4-methylphenyl-N-[4-(2-ethyl-4-(3-methylphenyl)-1,3-thiazol-5-yl)-pyridine-2-yl]-sulfoximine

¹H NMR [CDCl₃, 300 MHz] 1.41-1.46 (3H, t, J=7.44 Hz); 2.31(3H, s); 2.44 (3H, s); 3.03-3.1 (2H, q, J=7.59 Hz); 3.34 (3H, s); 6.58-6.60 (1H, dd, J=1.62Hz, J= 5.37 Hz); 6.88 (1H, s); 7.09-7.21 (3H, m); 7.33-7.38 (3H, t, J=8.02Hz); 7.87-7.90 (2H, d, J= 8.31 Hz); 7.93-7.95 (1H, d, J=5.4Hz). Yield =62 %.

### EXAMPLE 16

### Di-n-butyl-N-[4-(2-ethyl-4-(3-methylphenyl)-1,3-thiazol-5-yl)-pyridine-2-yl]-sulfoximine

¹H NMR [CDCl₃, 300 MHz] 0.93-0.98 (6H, t, J=7.33 Hz); 1.41-1.46 (4H, m); 1.48-1.53 (3H, t, J=7.39Hz); 1.78-1.86 (4H, m); 2.32 (3H, s); 3.03-3.10 (2H, q, J= 7.56 Hz); 3.40-3.47 (4H, m); 6.58-6.61(1H, dd, J=1.54 Hz, J=5.37 Hz); 6.80 (1H, s); 7.09-7.21 (3H, m); 7.40 (1H, s); 7.99-8.01(1H, d, J=5.37Hz). Yield =44 %.

### EXAMPLE 17

### S-Methyl-S-(3-chloro-4-fluro-phenyl)-N-[4-(2-ethyl-4-(3-methylphenyl)-1,3-thiazol-5-yl)-pyridine-2-yl]-sulfoximine

¹H NMR [CDCl₃, 300 MHz] 1.41-1.46 (3H, t, J=7.54Hz), 2.32 (3H, s), 3.03-3.11(2H, q, J= 7.57Hz ), 3.36( 3H, s ), 6.63-6.65( 1H, dd, J=1.56Hz, J=5.37Hz ), 6.87(1H, s ), 7.12- 7.18( 3H, m ), 7.28-7.33( 1H, m ); 7.39( 1H, s ), 7.90-7.94( 2H, m ), 8.08-8.11(1H, dd, J=2.34Hz, J=6.66Hz ). Yield =26 %.

### EXAMPLE 18

### 1-Imino-N-[4-(2-ethyl-4-(3-methylphenyl)-1,3-thiazol-5-yl)-pyridime-2-yl]-tetrahydro-2H-thiopyran-1-oxide

¹H NMR [CDCl₃, 300 MHz] 1.41-1.46( 3H, t, J=7.56Hz ); 1.64-1:69( 2H, m ); 2.01-2.1( 4H, m ); 2.33 ( 3H, s ); 3.03-3.11( 2H, q , J=7.57Hz ); 3.28-3.37(2H, m ); 3.65-3.68( 2H, m );6.60-6.63( 1H, dd, J=1.59Hz, J=5.34Hz ); 6.80( 1H, s ); 7.10=7.24( 3H, m); 7.41( 1H, s)8.01-8.02( 1H, d, J=5.43Hz ). Yield =40 %.

### EXAMPLE 19

### 1=Imino-N-[4-(2-ethyl-4-(3-methylphenyl)-1,3-thiazol-5-yl)-pyridine-2-yl]-tetrahydro-thiophene-1-oxide

¹H NMR [CDCl₃, 300 MHz] 1.31-1.36(3H, t, J=7.51Hz ); 2.05-2.1 2H, m );2.12-2.18( 2H, m ); 2.28 ( 3H, s ); 2.98-3.06(2H, q , J=7.54Hz ); 3.24-3.29( 2H, m ); 3.47-3.53( 2H, m ); 6.58( 1H, s ); 6.61-6.64(1H, dd,J=1.47Hz, J=5.33Hz ); 1,16-7.25( 3H, m ); 7.35(1H, s) 8.03-8.05( 1H, d, J=5.31Hz). Yield =71 %.

### EXAMPLE 20

### (-)-(S)-Methyl-S-Phenyl-N-[4-(2-ethyl-4-phenyl)-1,3-thiazole-5=yl)-pyridine-2-yl]-sulfoximine

¹H NMR [CDCl₃, 300Hz] 1.43(3H, t, J = 7.5Hz); 3.06(2H,q, J = 7.56 Hz); 3.35(3H,s); 6.61(1H, dd, J=1.38Hz & 5.31Hz); 6.88(1H,s); 7.3(3H,m); 7.4(2H,m); 7.5(3H,m); 7.6(3H,dd, J=7.07 & 15.5 Hz). Yield =33 %.

### EXAMPLE 21

### (+)-S-Methyl-S=Phenyl-N-[4-(2-ethyl-4-phenyl)-1,3-thiazole-5-yl)-pyridine-2-yl]-sulfoximine

¹H NMR [CDCL₃, 300Hz] 1.43 (3H, t, J = 7.5Hz); 3.06(2H,q, J = 7.56 Hz); 3.35(3H,s); 6.61(1H, dd, J=1.38 Hz & 5.31Hz); 6.88(1H,s); 7.3(3H,m); 7.4(2H,m); 7.5(3H,m); 7.6(3H,dd, J=7.01 & 15.5Hz). Yield =29 %.

### EXAMPLE 22

### S-Isopropyl-S-Phenyl-N-[4-(2-ethyl-4-phenyl)-1,3-thiazole-5-yl)-pyridine-2-yl]-sulfoximine

¹H NMR [CDCl₃, 300 Hz] 1.2 (3H, d, J =6.8Hz); 1.4(6H,m); 3.08(2H, q, J=7.5Hz), 3.6 (1H, m) 6.5(1H, d, J=5.356 Hz); 6.8(1H, d, J=7.35Hz), 7.4(4H, m), 7.5(4H, m); 7.8(2H, dd, J=7.7&7.1Hz) Yield =17 %.

### EXAMPLE 23

### S-(4-Methoxy-phenyl)-S-methyl- N-[4-(2-ethyl-1-4-phenyl)-1,3-thiazole-5-yl)-pyridine-2-yl]-sulfoximine

¹H NMR [CDCl₃, 300Hz] 1.43 (3H, t, J = 7.5Hz); 3.06(2H,q, J = 7.56 Hz); 3.35 (3H,s); 3.87(3H,s); 6.61(1H,m); 6.88(1H,s); 7.09(2H,dd); 7.26(3H,m); 7.38(1H,s); 7.92 (3H,m) Yield =35 %.

### EXAMPLE 24

### S-Methyl-S-(3-Methylphenyl)-N-[4-{2-ethyl-4-phenyl-thiazol-5-yl}-pyridine]-sulfoximine

¹H NMR [CDCl₃, 300 Hz] 1.4(3H, t, J=7.56 Hz); 2.04 (3H,s); 3.08(2H, q, J=7.56Hz); 3.3(3H,s), 6.6(1H, d, J= 5.2 Hz); 6.8(1H,s); 7.2(4H,m); 7.4(3H,m); 7.6(2H,m) 7.9(1H,d, J=5.4Hz) Yield =13 %.

### EXAMPLE 25

### S-Methyl-S-(3-fluorophenyl)-N-[4-{2-ethyl-4-phenyl-thiazol-5-yl}-pyrtdine]-sulfoximine

¹H NMR [CDCl₃, 300Hz] 1.4(3H, t, J=7.56Hz); 3.08 (2H, q, J=7.56Hz); 3.3(3H,s) 6.6(1H,dd, J=1.5Hz & 3.17 Hz); 6.8(1H,s); 7.3(4H,m); 7.4(2H,m); 7.5(1H,d,J=5.25Hz) 7.7(1H,s); 7.8(1H,d, J=8.01Hz); 7.9(1H,d, J=5.4Hz). Yield =35 %.

### EXAMPLE 26

### 4-[2-ethyl-4-phenyl-thiazol-5-yl]-2-(Dicyclohexyl suifoximine)-pyridine

¹H NMR [CDCl₃, 300 MHz] 1.25(6H,m); 1.4(3H, t, J = 7.5Hz); 1.64(3H,m); 1.68(3H,m); 1.72(2H,m) 1.89(4H,broad); 2.17(4H,m); 3.05(2H, q, J= 7.7 Hz); 6.5(1H, dd, J= 1.53 & 3.8 Hz); 6.9(1H,s) 7.2(3H,m); 7.5(2H,m); 8.03(1H, d, J = 5.4Hz). Yield =29%.

### EXAMPLE 27

### S-Methyl-S-(4-fluorophenyl-N-[4-{2-ethyl-4-phenyl-thiazol-5-yl}-pyridine]-sulfoximine

¹H NMR [CDCl₃, 300 MHz] 8.05 (m, 2H), 7.96 (d, 1H, J=5.3 Hz), 7.5(m, 2H), 7.31(m, 3H), 7.24(m, 2H), 6.87(s, 1H), 6.64(dd, 1H, J=1.6, 5.3Hz), 3.37(s, 3H), 3.12(q, 2H, J=7.6Hz), 1.47(t, 3H, J=7.6Hz). Yield =28 %.

### EXAMPLE 28

### S-Cyclopentyl-S-phenyl-N-[4-{2-ethyl-4-phenyl-thiazol-5-yl}=pyridine]-sulfoximine

¹H NMR [CDCl₃, 300MHz] 7.91-7.88 (m, 3H), 7.61-7.52 (m, 6H), 7.29(s, 2H), 6.87(s, 1H), 6.54(dd, 1H, J=1.6, 5.3Hz), 3.8(t, 1H, J=8.0Hz), 3.1(q, 2H, J=7.5Hz), 2.29(m, 1H), 2.18(m, 1H), 1.75(m, 3H), 1.46(t, 3H, J=7.5Hz). Yield =41 %.

### EXAMPLE 29

### (-)-S-Cyclopentyl-S-phenyl-N-[4-(2-ethyl-4-phenyl)-1,3-thiazol-5-yl)-pyridine-2-yl]-sulfoximine

¹H NMR, [CDCl₃, 300 MHz] 1.40-1.46(3H, t, J=7.55Hz), 1.73-1.75(5H, m ), 2.01-2.17( 2H, m ), 2,28-2.31 ( 1H, m ), 3.02-3.10( 2H, q, J=7.57Hz ), 3.75-3.80( 1H, m ), 6.53-6.55(1H, d, J=4.92Hz ), 6.88( 1H, s ), 7.28-7.29( 2H, m ), 7.44-7.48( 2H, m ), 7.49-7.60( 4H, m ), 7.89-7.91 ( 3H, m ). Yield =50 %.

### EXAMPLE 30

### (+)-S-Cyclopentyl-S-phenyl-N-[4-(2-ethyl-4-phenyl)-1,3-thiazol-5-yl)-pyridine-2-yl]-sulfoximine

¹H NMR, [CDCl₃, 300 MHz] 1.40-1.46(3H, t, J=7.55Hz), 1.73-1.75(5H, m), 2.01-2.17(2H, m), 2.28-2.31(1 H, m), 3.02-3.10(2H, q, J=7.57Hz ), 3.75-3.80( 1H, m ), 6.53-6.55(1H, d, J=4.92Hz ), 6.88( 1H, s ), 7.28-7.29( 2H, m ), 7.44-7.48( 2H, m ), 7.49-7.60( 4H, m ), 7.89-7.91 ( 3H, m ). Yield =39 %.

### EXAMPLE 31

### S-methyl-S-phenyl-N-[4-(2-ethyl-4-phenyl)-1,3-thiazol-5-yl)-pyridine-2-yl]-sulfoximine

¹H NMR [CDCl₃, 300 MHz] 1.41-1.46(3H, t, J=7.56 Hz), 3.03-3.11(2H, q, J=7.56Hz), 3.37(3H, s), 6.59-6.61(1H, dd, J=1.57Hz, J=5.35 Hz), 6.89(1H, s), 7.29-7.31(3H, m), 7.48-7.50 (2H, m), 7.56-7.61 (3H, m), 7.93-7.95(1H, d, J=5.42Hz), 8.00-8.03 (2H, dd, J=1.58Hz, J=7.69Hz). Yield =67 %.

### EXAMPLE 32

### S,S-diphenyl-N-[4-(2-ethyl-4-phenyl-thiazol-5-yl)-pyridin-2-yl]-sulfoximine

¹H NMR[CDCl₃, 300 MHz] 1.42 (t, 3H, J=7.59 Hz), 3.04 (q, 2H, J=7.51 Hz), 6.58 (d, 1H, J=5.49 Hz), 7.04 (s, 1H), 7.47 (m, 10H), 7.91 (d, 1H, J=5,1 Hz), 8.03 (d, 5H, J=6.38 Hz). Yield =63 %.

### EXAMPLE 33

### (-)-S-Methyl-S-phenyl-N-{4-[2-ethyl-4-(4-fluoro-phenyl)-thiazol-5-yl]-pyridin-2-yl}-

### sulfoximine

¹H NMR[CDCl₃, 300 MHz] 7.9(m, 3H), 7.6(m, 3H), 7.4(m, 2H), 6.9(t, 2H, J=8.8, 8.7Hz), 6.8(d, 1H, J=0.6Hz), 6.6(dd, 1H, J=1.5, 3.9Hz), 3.3(s, 3H), 3.0(q, 2H, J=7.5, 7.8Hz), 1.4(t, 3H). Yield =76 %.

### EXAMPLE 34

### S-(4-Methoxy-phenyl)-S-methyl-N-{4-[2-ethyl-4-(4-fluoro-phenyl)-thiazol-5-yl]-pyridin-2-yl}-sulfoximine

¹H NMR, [CDCl₃, 300 MHz] 8.0(d, 1H, J=5.4Hz), 7.9(d, 2H, J=9Hz), 7.4(m, 2H), 7.0(m, 4H), 6.8(s, 1H), 6.5 (dd, 1H, J=1.5, 3.9Hz), 3.8(s, 3H), 3.3(s, 3H), 3.0(q, 2H, J=7.5, 7.5Hz), 1.4(t, 3H, J=7.8, 7.5 Hz). Yield =57 %.

### EXAMPLE 35

### S-Isopropyl-S-phenyl-N-{4-[2-ethyl-4-(4-fluoro-phenyl)-thiazol-5-yl]-pyridin-2-yl}-sulfoximine

¹H NMR, [CDCl₃, 300 MHz] 7.9 (d, 1H, J=5.4Hz), 7.8(d, 2H, J=6.9Hz), 7.6(d, 1H, J=7.2Hz), 7.5(t, 2H, J=7.2, 7.5Hz), 7.4(m, 2H), 6.9(t, 2H, J=8.7, 9Hz), 6.8(s, 1H), 6.5(dd, 1H, J=1.5, 3.9Hz), 3.6(m, 1H), 3.0(q, 2H, J=7.5, 7.5Hz),1.4(t, 6H, J=7.5, 7.5Hz), 1.2(d, 3H, J=6.9Hz). Yield =45 %.

### EXAMPLE 36

### (-)-Isopropyl-S-phenyl-N-{4-[2-ethyl-4-(4-fluoro-phenyl)-thiazol-5-yl]-pyridin-2-yl}-sulfoximine

¹H NMR, [CDCl₃, 300 MHz] 7.9(d, 1H, J=5.4Hz), 7.8(d, 2H, J=6.9Hz), 7.5(d, 1H, J=7.8Hz), 7.4(m, 4H), 6.9(t, 2H, J=9, 8.7Hz), 6.8(d, 1H, J=0.9Hz), 6,5(t, 1H, J=1.5, 5.4Hz), 3.6(m, 1H), 3.0(q, 2H, J=7.8, 7.5Hz), 1.4(t, 6H, J=7.5, 7.2Hz), 1.2(d, 3H, J=6.9Hz). Yield =45 %.

### EXAMPLE 37

### (+)-S-Isopropyl-S-phenyl-N-{4-[2-ethyl-4-(4-fluoro-phenyl)-thiazol-5-yl]-pyridin-2-yl}-sulfoximine

¹H NMR, [CDCl₃, 300 MHz] 7.9(d, 1H, J=5.4Hz), 7.8(d, 2H, J=6.9Hz), 7.5(d, 1H, J=7.8Hz), 7.4(m, 4H), 6.9(t, 2H, J=9, 8.7Hz), 6.8(d, 1H, J=0.9Hz), 6.5(t, 1H, J=1.5, 5.4Hz), 3.6(m, 1H), 3.0(q, 2H, J=7.8, 7.5Hz), 1.4(t, 6H, J=7.5, 7.2Hz), 1.2(d, 3H, J=6.9Hz). Yield =57 %.

### EXAMPLE 38

### S-Ethyl-S-phenyl-N-{4-[2-ethyl-4-(4-fluoro-phenyl)-thiazol-5-yl]-pyridin-2-yl}-sulfoximine

¹H NMR[CDCl₃, 300 MHz] 7.9(m, 3H), 7.5-7.6(m, 4H), 7.4(dd, 2H, J=5.4, 3.3Hz), 6.9(t, 2H, J=8.7, 8.7Hz),6.85(s, 1H), 6.5(dd, 1H, J=1.8, 3.6Hz), 3.4(m, 2H), 3.0(q, 2H, J=7.5, 7.5Hz),1.4(t, 3H, J=7.5, 7.8Hz), 1.27(t, 3H, J=7.5, 7.5Hz). Yield =48 %.

### EXAMPLE 39

### S-Methyl-S-phenyl-N-[4-(2-ethyl-4-(4-fluoro-phenyl)-1,3-thiazol-5-yl)-pyridine-2-yl]-sulfoximine

¹H NMR, [CDCl₃, 300 MHz] 1.41-1.46(3H, t, J=7.57 Hz), 3.02-3.10(2H, q, J=7.57 Hz), 3.37(3H, s), 6.58-6.61(1H, dd, J=1.54Hz, J=5.33Hz), 6.85(1H, s), 6.95=7.01(2H, m), 7.44-7.49( 2H, m ), 7.54-7.64( 3H, m ), 7.96-8.02( 3H, m ) Yield =82 %.

### EXAMPLE 40

### (+)-S-Methyl-S-phenyl-N-[4-(2-ethyl-4-(4-fluoro-phenyl)-1,3-thiazol-5-yl)-pyridine-2-yl]-sulfoximine

¹H NMR [CDCl₃, 300 MHz] 1.41-1.46 (3H, t, J=7.55Hz), 3.02-3.10(2H, q, J=7.53Hz, 3.37(3H, s), 6.59-6.61(1H, d, J=5.18Hz), 6.85(1H, s), 6.95-7.01(2H, t, J=8.66Hz), 7.44-7.49 (2H, m), 7.54-7.63(3H, m ), 7.96-8.02( 3H, m). Yield =38 %.

### EXAMPLE 41

### S-Methyl-S-4-fluoro-phenyl-N-[4-(2-ethyl-4-(4-fluoro-phenyl)-1,3-thiazol-5-yl)-pyridine-2-yl]-sulfoximine

¹H NMR [CDCl₃, 300 MHz] 1.41-1.46(3H, t, J=7.51Hz), 3.02-3.10(2H, q, J=7.50Hz), 3.37(3H, s), 6.60-6.62(1H, d, J=4.74Hz), 6.84(1H, s), 6.96-7.02(2H, t, J=8.55Hz), 7.21-7.24(2H, m), 7.45-7.49(2H, m), 7.96-8.02 (3H, m). Yield =78 %.

### EXAMPLE 42

### S-Cyclopentyl-S-Phenyl-N-[4-(2-ethyl-4-(4-fluorophenyl)-1,3-thiazol-5-yl)-pyridine-2-yl]-sulfoximine

¹H NMR [CDCl₃, 300 MHz] 1.40-1.45(3H, t, J=7.56Hz), 1.65-1.74(5H, m), 2.0-2.12 (2H, m), 2.29-2.33(1H, m), 3.01-3.09(2H, q, J=7.56Hz ), 3.74-3.79(1H, m ),6.52-6.54(1H, dd, J=1.48Hz, J=5.25Hz ); 6.83( 1H, s ); 6.91-6.97(2H, t, J=8.75Hz ),7.41-7.45( 2H, m ); 7.51-7.59( 3H, m ); 7.87-7.93( 3H, m ). Yield =71 %.

### EXAMPLE 43

### (-)-S-Methyl-S-4-fluoro-phenyl-N-[4-(2-ethyl-4-(4-fluoro-phenyl)-1,3-thiazol-5-yl)-pyridine-2-yl]-sulfoximine

¹H NMR [CDCl₃, 300 MHz] 1.41-1.46 (3H, t, J=7.54Hz), 3.02-3.10 (2H, q, J=7.58Hz), 3.37(3H, s), 6.61-6.62(1H, d, J=3.99Hz), 6.84(1H, s), 6.96-7.02(2H, t, J=8.71 Hz), 7.21-7.24(2H, m), 7.45-7.49(2H, m), 7.96-8.02(3H, m). Yield =74 %.

### EXAMPLE 44

### (+)-S-Methyl-S-4-fluoro-phenyl-N-[4-(2-ethyl-4-(4-fluoro-phenyl)-1,3-thiazol-5-yl)-pyridine-2-yl]-sulfoximine

¹H NMR [CDCl₃, 300 MHz] 1.41-1.46(3H, t, J=7.54 Hz), 3.02-3.10 (2H, q, J=7.58 Hz), 3.37 (3H, s), 6.61-6.62(1H, d, J=3.99Hz), 6.84( 1H, s ),6.96-7.02( 2H, t, J=8.71Hz ),7.21-7.24( 2H, m ), 7.45-7.49( 2H, m ), 7.96-8.02( 3H, m ). Yield =75%.

### EXAMPLE 45

### S,S-Diphenyl-N-[4-(2-ethyl-4-(4-fluorophenyl)-thiazol-5-yl)-pyridin-2-yl]-sulfoximine

¹H NMR [CDCl₃, 300 MHz] 1.41 (t, 3H, J=7.59 Hz), 3.03 (q, 2H, J=7.59 Hz), 6.59 (d, 1H, J=5.31 Hz), 6.91 (m, 3H), 7.43 (m, 9H), 7.94 (d, 1H, J=5.37 Hz), 8.03 (m, 3H). Yield = 7 %.

### EXAMPLE 46

### S-Methyl-S-3-methylphenyl-N-[4-(2-ethyl-4-fluorophenyl-thiazol-5-yl)-pyridine]-sulfoximine

¹H NMR [CDCl₃, 300 MHz] 1.4(3H, t, J=7.56Hz); 2.4(3H,s); 3.07(2H, q, J=7.56Hz); 3.36(3H,s); 6.6(1H,dd, J=1.5Hz & 3.9Hz); 6.8(1H,s); 6.9(2H,m); 7.4(4H,m); 7.7(1H,m); 7.8(1H,s); 8.0(1H,d, J=5.4Hz) Yield =49 %.

### EXAMPLE 47

### S-Methyl-S-3-fluorophenyl-N-[4-{2-ethyl-4-(4-fluorophenyl)-thiazol-5-yl}-pyridine-2yl]-sulfoximine

¹H NMR [CDCl₃, 300 MHz] 1.4(3H, t, J=7.56Hz); 3.05(2H,q, J=7.56Hz); 3.3(3H,s); 6.6(1H, dd, J=1.5Hz & 3.9Hz); 6.8(1H,s); 6.9(2H,m); 7.3(1H,m); 7.4(2H,m); 7.5(1H,m)
7.7(1H,m); 7.8(1H,m); 8.0(1H,d, J=5.4Hz). Yield = 45 %.

### EXAMPLE 48

### S-Cyclohexyl-S-phenyl-N-{4-[2-ethyl-4-(4-fluorophenyl)-thiazol-5-yl]-pyridine}-sulfoximine

¹H NMR [CDCl₃, 300 MHz] 1.17(1H, m); 1.2(2H, m); 1.4(3H, t, J= 7.59Hz); 1.6(2H,m); 1.8(2H,m) 2.00(1H,m); 2.35(1H,m); 3.01(2H, q, J= 7.576Hz); 3.2(1H,m); 3.5((1H,d, J=4.74Hz); 6.53(1H, dd, J= 1.41 Hz & 3.9Hz); 6.56(1H,s); 6.9(2H,m); 7.44(2H,m); 7.52(2H,m); 7.6(1H,m); 7.8(2H, d, J=7.14Hz); 7.9(1H, d, J=5.25Hz).
Yield =24 %.

### EXAMPLE 49

### S-Methyl-S-phenyl-N-[4-(2-ethyl-4-m-benzoic acid-thiazol-5-yl)-pyridine-2-yl]-sulfoximine

S-Methyl-S-phenyl-N-{3-(2-ethyl-4-m-benzoic acid ethyl ester)-thiazol-5-yl-pyridine-2-yl}-sulfoximine (0.205g) was dissolved in THF & MeOH at ambient temperature, LiOH.H₂O (0.035g) dissolved in water was added to the reaction mixture and stirred at ambient temperature for 2 to 4 hours The reaction mixture was basified to pH 8 by adding solution of sodium bicarbonate. The aqueous layer was extracted with ethyl acetate, organic layer collected, dried over sodium sulfate & evaporated under table vaccum. The crude compound (0.120g) was purified by flash column chromatography. ¹H NMR[CDCl₃, 300 MHz] 1.45(3H, t, J=7.54Hz), 3.3 (3H,s); 6.6(1H, dd, J= 5.4Hz); 6.8(1H,s); 7.4(1H,m),7.6(4H,m); 7.9(4H,m):8.2(1H,s). Yield=26 %.

### EXAMPLE 50

### S-Methyl-S-phenyl-N-[4-(2-ethyl-4-(3-fluoro-phenyl)-1,3-thiazol-5-yl)-pyridine-2-yl]-sulfoximine

¹H NMR [CDCl₃, 300 MHz] 1.41-1.46(3H, t, J=7.54Hz), 3.03-3.10(2H, q, J=7.54Hz ), 3.33-3.37(3H, s), 6.60-6.62(1H, dd, J=1.56Hz, J=5.35Hz), 6.86-6.87(1H, d, J=0.93Hz) 6.96-7.02(1H, m), 7.22-7.25(2H, m), 7.54-7.63(3H, m), 7,97-8.02(3H, m). Yield = 49 %.

### EXAMPLE 51

### S-Isopropyl-S-phenyl-N-[4-(2-ethyl-4-(3-fluoro-phenyl)-1,3-thiazol-5-yl)-pyridine-2-yl]-sulfoximine

¹H NMR [CDCl₃, 300 MHz] 7.9 (d, 1H, J=5.4Hz), 7.8(d, 2H, J=6.9Hz), 7.6(d, 1H, J=7.2Hz), 7.5(t, 2H, J=7.2, 7.5Hz), 7.4(m, 2H), 6.9(t, 2H, J=8.7, 9Hz), 6.8(s, 1H), 6.5(dd, 1H, J=1.5, 3.9Hz), 3.6(m, 1H), 3.0(q, 2H, J=7.5, 7.5Hz), 1.4(t, 6H, J=7.5, 7.5Hz), 1.2(d, 3H, J=6.9Hz). Yield = 58 %.

### EXAMPLE 52

### S-Cyclopentyl-S-phenyl-N-[4-(2-ethyl-4-(3-fluoro-phenyl)-1,3-thiazol-5-yl)-pyridine-2-yl]-sulfoximine

¹H NMR, [CDCl₃, 300 MHz) 1.40-1.46(3H, t, J=7.59Hz), 1.68-1.75(3H, m),1.98-2.10( 2H.m), 2.29-2.33(1H, m), 3.02-3.09(2H, q), 3.71-3.79(1H, m), 6.54-6.56(1H, d, J=5.31Hz)6.84(1H, s), 6.96-6.97(1H, m), 7.20-7.23(3H, m), 7.49-7.59(3H, m), 7.88-7.94(3H, m). Yield =53 %.

### EXAMPLE 53

### S-Methyl-S-phenyl-N={4-[2-ethyl-4-naphthalene-1-yl-thiazol-5-yl]-pyridin-2-yl}-sulfoximine

¹H NMR [CDCl₃, 300 MHz] 7.9(m, 4H), 7.7(t, 2H, J=3.6, 5.4Hz), 7.54(s, 1H), 7.51 (t, 2H, J=6.3, 1.5Hz), 7.4(m, 4H), 6.3(d, 1H, J=1.2Hz), 6.2(dd, 1H, J=3.6, 1.8Hz), 3.2(s, 3H), 3.1(q, 2H, J=7.5, 7.8Hz), 1.2(t, 3H, J=2.1, 3.1Hz). Yield =56 %.

### EXAMPLE 54

### S-Methyl-S-phenyl-N-{4-[2-ethyl-4-(3-trifluoromethylphenyl)-thiazol-5-yl]-pyridin-2-yl}-sulfoximine

¹H NMR [CDCl₃, 300 MHz] 8.02(d, 3H, J=6.9Hz), 7.85(s, 1H), 7.66(t, 2H, J=7.1Hz), 7.59(t, 3H, J=7.4Hz), 7.43(t, 1H, J=7.7Hz), 6.85(s, 1H), 6.6(d, 1H, J=4.3Hz), 3.36(s, 3H), 3.12(q, 2H, J=7.5Hz), 1.48(t, 3H, J= 7.5Hz). Yield =52 %.

### EXAMPLE 55

### S-Methyl-S-phenyl-N-{4-[2-ethyl-4-(4-methoxyphenyl)-thiazol-5-yl]-pyridin-2-yl}-sulfoximine

¹H NMR [CDCl₃, 300 MHz] 8.04(m, 2H), 7.56(d, 2H, J=1 1.3Hz), 7.59(m, 3H), 7.25(m, 2H), 6.95(m, 2H), 6.79(m, 2H), 3.84(s, 3H), 3.33(m, 4H), 1.96(m, 2H), 1.27(m, 3H). Yield =40 %.

### EXAMPLE 56

### S-Methyl-S-phenyl-N-{4-[2-(4-methylsulfanyl-phenyl)-4-m-tolyl-thiazol-5-yl]-pyridin-2-yl}-sulfoximine

¹H NMR [CDCl₃, 300 MHz] 8.0(d, 2H, J=7.2Hz), 7.9(m, 3H), 7.5(m, 4H), 7.4(s, 1H), 7.3(s, 2H), 1.1(m, 2H), 6.9(s, 1H), 6.6(dd, 1H, J=1.5, 3.9Hz), 3.3(s, 3H), 2.5(s, 3H), 2.3(s, 3H). Yield =67 %.

### EXAMPLE 57

### S-Methyl-S-phenyl-N-{4-[2-(4-methylsulfinyl-phenyl)-4-m-tolyl-thiazol-5-yl]-pyridin-2-yl}-sulfoximine

A round bottom flask containing S-methyl-S-phenyl-N-{4-[2-(4-methylsulfanyl-phenyl)-4-m-tolyl-thiazol-5-yl]-pyridin-2-yl} -sulfoximine (0.250g) in DMF was cooled. To this was added metacholoroperbenzoic acid (0.163g) & stirred at ambient temperature for few hrs. After completion of the reaction, the reaction mixture was extracted with ethyl acetate.The organic layer was separated, dried & evaporated to get brown oil. The crude was purified by flash column chromatography to give pale yellow solid (0.079 g).

¹H NMR [CDCl₃, 300 MHz] 8.1(d, 2H, J=8.4Hz), 7.9-8.0(m, 3H), 7.7(d, 2H, J=8.4Hz), 7.6(m, 3H), 7.4(s, 1H), 7.28(s, 1H), 7.21(m, 2H), 6.9(d, 1H, J=0.9Hz), 6.6(t, 1H, J=1.5, 3.9Hz), 3.39(s, 3H), 2.79(s, 3H), 2.35(s, 3H). Yield =29 %.

### EXAMPLE 58

### S-Methyl-S-phenyl-N-{4-[2-ethyl-4-(3,4-difluorophenyl)-thiazol-5-yl]-pyridin-2-yl}-sulfoximine

¹H- NMR[CDCl₃, 300 MHz] 8.02 (m, 3H), 7.64-7,54(m, 3H), 1.20-1.04(m, 2H), 6.84(m, 1H), 6.63(dd, 1H, J=1.6, 5.4Hz), 3.37(s, 3H), 3.09-3.01 (q, 2H, J=7.6Hz), 1.46(t, 3H, J=7.6Hz). Yield =78 %.

### EXAMPLE 59

### S-Methyl-S-(4-fluorophenyl)-N-{4-[2-ethyl-4-(3,4-difluorophenyl)-thiazol-5-yl]-pyridin-2-yl}-sulfoximine

¹H NMR[CDCl₃, 300 MHz] 8.04(m, 3H), 7.36-7:31 (m, 1H), 7.24-7.21 (m, 2H), 7.09(m, 1H), 6.83(s, 1H), 6.65(dd, 1H, J=1.4, 5.3Hz), 3.37(s, 3H), 3.10(q, 2H, J=7.6Hz), 1.46 (t, 3H, J=7.6Hz). Yield =68 %.

### EXAMPLE 60

### S-Isopropyl-S-phenyl-N-{4-[2-ethyl-4-(3,4-difluorophenyl)-thiazol-5-yl]-pyridin-2-yl}-sulfoximine

¹H NMR[CDCl₃, 300 MHz] 8.00 (d, 1H, J=5.3Hz), 7.88(d, 2H, J=7.3Hz), 7.62(m, 3H), 7.17(d, 1H, J=2.3Hz),7.06(q, 1H, J=9.2Hz), 6.83(s, 1H), 6.59(d, 1H, J=4.0Hz), 3.65-3.54(m, 1H), 3.09(q, 2H, J=7.5Hz),1.45-1.4(m, 6H), 1.27(t, 3H, J=1.5Hz). Yield =45 %.

### EXAMPLE 61

### S-Isopropyl-S-phenyl-N-{4-[2-ethyl-4-(3,5-difluorophenyl)-thiazol-5-yl]-pyridin-2-yl}-sulfoximine

¹H NMR [CDCl₃, 300 MHz] 7.9 (d, 1H, J=5.1Hz), 7.8(d, 2H, J=7.2Hz), 7.5-7.6 (m, 3H), 7.0(d, 2H, J=6.3Hz), 6.8(s, 1H), 6.7(m, 1H), 6.5(t, 1H, J=1.2, 3.9Hz), 3.6(m, 1H), 3.0(q, 2H, J=7.5, 7.5Hz), 1.4(t, 6H, J=7.5, 7.5Hz), 1.1(d, 3H, J=6Hz). Yield =51 %.

### EXAMPLE 62

### S-Methyl-S-phenyl-N-{4-[2-ethyl-4-(3,5-difluorophenyl)-thiazol-5-yl)-pyridin-2-yl}-sulfoximine

¹H NMR [CDCl₃, 300 MHz] 1.4(3H,t, J = 7.5Hz); 3.04(2H, q, J = 7.56 Hz); 3.3(3H,s); 6.61(1H,dd,J=1.56Hz & 3.75Hz); 6.73(1H,s); 6.8(1H,m); 7.03(2H,dd,J=2.27&6.19Hz) 7.6(3H,m), 7.9(3H,m). Yield =70 %.

### EXAMPLE 63

### S,S-Diphenyl-N-{4-[2-ethyl-4-(3,5-difluorophenyl)-thiazol-5-yl]-pyridin-2-yl}-sulfoximine

¹H NMR [CDCl₃, 300 Hz] 8.0(m, 5H), 7.46-7,52(m, 6H), 7.0(br, 3H), 6.6(m, 2H), 3.0(q, 2H, J=7.5, 7.5Hz), 1.4(t, 3H, J=7.5, 7.8Hz). Yield =73 %.

### EXAMPLE 64

### S-Methyl-S-phenyl-N-{4-[2-ethyl-4-p-tolyl-thiazol-5-yl)-pyridin-2-yl}-sulfoximine

¹H NMR [CDCl₃, 300 MHz] 8.04(d, 1H, J=7.1Hz), 7.95(d, 1H, J=5.3Hz), 7.63-7.64(m, 3H), 7.40(d, 2H, J=8 Hz), 7.12(d, 2H, J=7.9Hz), 6.90(s, 1H), 6.0(dd, 1H, J=1.4, 5.3Hz), 3.38(s, 3H), 3.10(q, 2H, J=7.6Hz), 2.35(s, 3H), 1.46(t, 3H, J=7.6Hz). Yield =55 %.

### EXAMPLE 65

### S-Cyclopentyl-S-phenyl-N-{4-(2-(2,6-difluorophenyl)-4-(3-fluorophenyl)-(1,3)-thiazol-5-yl]-pyridin-2-yl}-sulfoximine

¹H NMR [CDCl₃, 300 MHz] 8.0(m, 3H), 7.93-7.53(m, 3H), 7.41-7.29(m, 2H), 7.10-6.96(m, 3H), 6.65(m, 1H), 3.79-3.76(m, 1H), 3.50-3.48(m, 1H), 2.33(m, 1H), 2.14-2.01(m, 2H), 1.75(m, 3H). Yield = 8 %.

### EXAMPLE 66

### S-Isopropyl-S-phenyl-N-{4-[2-(2,6-difluorophenyl)-4-(3-fluorophenyl)-(1,3)-thiazol-5-yl]-pyridin-2-yl}-sulfoximine

¹H NMR [CDCl₃, 300 MHz] 8.02(d, 1H, J=5.4Hz), 7.9-7.88(m, 2H), 7.65-7.52 (m, 3H), 7.46-7.29(m, 4H), 7.10-7.04(m, 2H), 7.01-6.96(m, 2H), 6.67(dd, 1H, J=1.5, 5.4Hz), 3.6(m; 1H), 1.4(d, 3H, J=7.5Hz), 1.1(d, 3H, J=7.5Hz). Yield =60 %.

### EXAMPLE 67

### S-Cycloheayl-S-phenyl-N-{4-[2-(2,6-difluorophenyl)-4-(3-fluorophenyl)-(1,3)-thiazol-5-yl]-pyridin-2-yl}-sulfoximine

¹H NMR [CDCl₃, 300 MHz] 8.01(d, 1H, J=5.2Hz), 7.88(d, 2H, J=7.5Hz), 7.61-1.51 (m, 3H), 7.41-7.24(m, 4H), 6.66(d, 1H, J= 5.4Hz), 3.3(m, 1H), 2.0(d, 1H), 1.8(m, 2H), 1.4(m, 2H), 1.2(m, 3H), 1.14(m, 2H). Yield =50 %.

### EXAMPLE 68

### S-Cyclohexyl-S-phenyl-N-{4-[2-(4-fluorophenyl)-4-(3-fluorophenyl)-(1,3)-thiazol-5-yl)-pyridin-2-yl}-sulfoximine

¹H NMR [CDCl₃, 300 MHz] 1.17(1H,m); 1.2(2H,m); 1.4(2H,m); 1.5(1H,m); 1.8(2H,m) 2.00(1H,m); 2.35(1H,m); 3.3(1H,m); 6.6(1H, dd, J= 5.2 Hz); 6.9(1H,s), 7.01(1H,m) 7.1(2H,m); 7.3(3H,m); 7.5(2H,m); 7.6(1H,m); 7.8(2H,m); 7.9(3H,m). Yield =50 %.

### EXAMPLE 69

### S-Isopropyl-S-phenyl-N-{4-[2-(4-fluorophenyl)-4-(3-fluorophenyl)-(1,3)-thiazol-5-yl]-pyridin-2-yl}-sulfoximine

¹H NMR [(DMSO - D₆), 300MHz] 8.00-7.96(m, 3H); 7.90(d, J = 7.3 Hz, 2H); 7.62-7.52(m, 3H); 7.33-7.25(m, 3H); 7.19(t, J = 8.6Hz); 6.9(dd, J = 1.3Hz), 5.3Hz); 3.6(quint, J=6.8Hz, 1H); 1.45(d, J = 6.8Hz); 1.28(d, J = 6.8 Hz, 3H). Yield =50 %.

### EXAMPLE 70

### S-Cyclopentyl-S-phenyl-N-[4-(3-fluorophenyl)-1,3-thiazol-5-yl)-pyridine-2-yl]-sulfoximine

H¹-NMR, [CDCl₃, 300 MHz] -1.57-1.63(2H, m), 1.65-1.77( 3H, m ), 1.98-2.13(2H, m ), 2.30-2.37(1H, m ), 3.75-3.83(1H, m), 6.57-6.59(1H, dd, J=1.41Hz, J=5.23Hz), 6.88(1H, s), 6.97-7.02(1H, m), 7.22-7.25( 3H, m), 7.49-7.59(3H, m), 7.88=7.91( 2H, d, J=7.11Hz), 7.95-7.97(2H, d, J=5.31Hz), 8.82(1H, s). Yield =85 %.

### EXAMPLE 71

### S-Methyl-S-phenyl-N-[4-(3-fluorophenyl)-1,3-thiazol-5-yl)-pyridine-2-yl] sulfoximine

H¹-NMR [CDCl₃, 300 MHz] 3.38(3H, s), 6.64-6.66(1H, d, J=5.27Hz), 6.90(1H, s), 6.99-7.05(1H, m), 7.23-7.29(3H, m), 7.55-7.66(3H, m), 8.00-8.03(3H, m), 8.84(1H, s). Yield =54 %.

### EXAMPLE 72

### S-Isopropyl-S-phenyl-N-[4-(3-fluorophenyl)-1,3-thiazol-5-yl)-pyridine-2-yl]-sulfoximine

H¹-NMR [CDCl₃, 300 MHz] 1.27-1.29(3H, d, J=6.81Hz), 1.41-1.44(3H, d, J=6.78Hz), 3.57-3.66(1H, m), 6.59-6.62(1H, dd, J=1.27, 5.19Hz), 6.89(1H, s), 6.97-7.02(1H, m), 7.23(2H, s), 7.27(1H, s), 7.51-7.61 (3H, m), 7.87-7.89 (2H, d, J=7.19z), 7.98-8.0( 1H, d, J=5.22 Hz), 8.83 (1H, s). Yield =75 %.

### EXAMPLE 73

### S-Methyl-S-phenyl-N-[4-(4-fluorophenyl)-1,3-thiazol-5-yl-pyridine-2-yl]-sulfoximine

H¹-NMR [CDCl₃, 300 MHz] 3.38(3H, s), 6.63-6.65(1H, d, J=5.22Hz), 6.89(1H, s), 6.97-7.03 (2H, t, J=8.61Hz), 7.48-7.59(4H, m), 7.62-7.66(1H, m), 8.00-8.03(3H, m), 8.83 (1H, s). Yield =82 %.

### EXAMPLE 74

### S-Isopropyl-S-phenyl-N-[4-(4-fluorophenyl)-1,3-thiazol-5-yl)-pyridine-2-yl]-sulfoximine

H¹-NMR [CDCl₃, 300 MHz] 1.27-1.29(3H, d, J=6.81Hz), 1.41-1.44(3H, d, J=6.78Hz), 3.57-3.66(1H, m), 6.58-6.59(1H, d, J=5.1Hz), 6.89(1H, s), 6.94-7.02 (2H, t, J=8.61Hz), 7.45-7.51(2H, m), 7.53-7.56 (2H, m), 7.60-7.65(1H, m), 7.87-7.89 (2H, d, J=7.47z), 7.98-7.99( 1H, d, J=5.3Hz), 8.82(1H, s). Yield =54 %.

### EXAMPLE 75

### S-Cyclopentyl=S-phenyl-N-[4-(4-fluorophenyl)-1,3-thiazol-5-yl)-pyridine-2-yl]-sulfoximine

H¹-NMR [CDCl₃, 300 MHz] 1.56-1.63(2H, m), 1.65-1.75(3H, m), 1.97-2.13(2H, m), 2.27-2.34(1H, m), 3.75-3.83( 1H, m), 6.56-6.58( 1H, dd, J=1.26Hz, 5.4Hz), 6.87(1H, s), 6.93-6.99( 2H, t, J=8.67Hz ), 7.44-7.51 ( 4H, m ), 7.54-7.62(1H, m), 7.88-7.91( 2H, d, J=7.32Hz), 7.95.7.97(1H, d, J=5,19Hz), 8.81(1H, s). Yield =45 %.

### EXAMPLE 76

### S-Methyl-S-phenyl-N[4-phenyl-(1,3)-thiazol-5-yl-pyridine-2-yl]-sulfoximine

H¹-NMR [CDCl₃, 300 MHz] 3.38(3H, s), 6.63-6.65(1H, dd, J=1.44Hz, J=5.25Hz), 6.92 (1H, s), 7.31-7.33(3H, m), 7.51-7.54(2H, m), 7.56-7.63(3H, m), 7.97-8.03( 3H, m) 8.84 (1H, s). Yield =41 %.

### EXAMPLE 77

### S-Isopropyl-S-phenyl-N-[4-phenyl-1,3-thiazol-5-yl)-pyridine-2-yl]-sulfoximine

H¹-NMR [CDCl₃, 300 MHz] 1.27-1.29(3H, d, J=6.79Hz), 1.41-1.43(3H, d, J=6.82Hz), 3.67(1H, m), 6.59-6.60(1H, d, J=5.33Hz), 6.91-6.92(1H, s), 7.29-7.31(3H, m), 7.49-7.56(5H, m), 7.87-7.9 (2H, d, J=8.53Hz), 7.94(1H, d), 8.83 (1H, s). Yield =40 %.

### EXAMPLE 78

### S-Cyclopenyl-S-phenyl-N-[(4-phenyl-thiazol-5-yl)-pyridine-2-yl]-sulfoximine

H¹-NMR[CDCl₃, 300 MHz] 1.61-1.77(5H, m), 1.98-2.05( 2H, m), 2.30-2.34(1H, m), 3.12-3.83(1H, m), 6.56-6.58(1H, dd, J=1.41Hz, J=5,28Hz), 6.90(1H, s), 7.26-7.3( 3H, m), 7.48-7.61(5H, m), 7.89-7.93(2H, m), 8.82(1H, s). Yield =36%.

### EXAMPLE 79

### Methane sulfonate salt of (+)-S-isopropyl,S-phenyl-N-{4-[2-ethyl-4-(4-fluorophenyl)-(1,3)-thiazol-5-yl]-pyridin-2-yl}-sulfoximine

To a solution of (+)-S-Isopropyl-S-phenyl-N-{4-[2-ethyl-4-(4-fluorophenyl)-(1,3)-thiazol-5-yl]-pyridin-2-yl}-sulfoximine (0.2g) in THF was added methanesulfonic acid (37mg) and stirred at ambient temperature for few hrs. The solvents were evaporated to yield solid compound (0.24g).
¹H NMR [CDCl₃, 300 MHz] 15.37(s,1H), 8.25(d, 1H, J=6,4Hz), 7.99(d, 2H, J=7.3 Hz), 7.75-7.63(m, 3H), 7.27(m, 2H), 7.05(s,1H), 6.94-6,85(m, 3H), 3.67-3.62(m, 1H), 3.09(q, 2H, J= 7.5Hz), 2.9(s, 3H), 1.51(d, 3H, J=6.7Hz), 1.45(t, 3H, J=7.5Hz), 1.29(d, 3H, J=6.7Hz). Yield =98 %.

### EXAMPLE 80

### N-Oxide of (+)-S-isopropyl,S-phenyl-N-{4-[2-ethyl-4-(4-fluorophenyl)-(1,3)-thiazol-5-yl]-pyridin-2-yl}-sulfoximine methane sulfonate salt

### Step 1: N-Oxide of(+)-S-isopropyl,S-pheny]-N-{4-[2-ethyl-4-(4-fluorophenyl)-(1,3)-thiazol-5-yl]-pyridin-2-yl}-sulfoximine

To a solution of (-)-S-isopropyl,S-phenyl-N-{4-[2-ethyl-4-(4-fluorophenyl)-(1,3)-thiazol-5-yl]-pyridin-2-yl}-sulfoximine(0.3g.) in dry chloroform was added m-chloroperbenzoic acid (0.344g) and the reaction mixture was heated to elevated temperature, for about 8 hrs. After completion, the reaction mixture was poured into sat. NaHCO3 solution & extracted by ethyl acetate. The organic layer was combined & dried over sodium sulfate, solvent was evaporated to yield brown oil (0.3g) .The crude product was purified by flash column chromatography.(0.15 g). Yield=48 %.

### Step 2: N-Oxide of (+)-S-isopropyl,S-phenyl-N-{4-[2-ethyl-4-(4-fluorophenyl)-(1,3)-thiazol-5-yl]-pyridin-2-yl}-sulfoximine methane sulfonate salt

The process was similar to that described in Example 70.
¹H NMR [CDCl₃, 300 MHz] 8.22(d, 1H, J=7.1Hz), 8.10(d, 2H, J=7.3 Hz), 7.76-7.68(m, 3H), 7.37(dd, 2H, J=5.4, 8.6Hz), 7.06(d. 1H, J=2.1Hz), 6.98(t, 2H, J=8.6Hz), 6.79(dd, 1H, J=2.2, 7.1Hz), 3.93(qui, 1H, J= 6.7Hz), 3.12(q, 2H, J=7.5Hz), 1.55(d, 3H, J=6.7Hz), 1.45(t, 3H, J=7.5Hz), 1.31(d, 3H, J=6.7Hz). Yield=67 %.

### Preparation of inorganic salts

To a stirred solution of sulfoximine in methanol was added methanolic acid such as methanolic hydrochoric, acid, methanolic sulphuric acid etc. The reaction mixture were stirred at ambient temperature for few hrs. The solvents were evaporated to yield solid compound.

### A) Demonstration of ex vivo efficacy of the compounds:

Blood was collected by venous puncture from 3 different volunteers in separate heparinized (100 IU/ml) tubes and incubated in the presence of 10 µM and 100 µM of test compounds for 1 hr at 37 °C. Following this LPS (1 ng/ml final concentration) was added and incubation continued for 5 hr. The reaction was terminated by placing the samples on ice for 10 min. The samples were then centrifuged, plasma separated and
stored at -70 °C until the analysis of TNF-α and IL-1β by ELISA:
The results of the finding are shown in table 1.

**Table 1: ex-vivo data**

| **Ex. No.** | **Ex-vivo(human whole blood)** | | |
|---|---|---|---|
| | **Conc (µM)** | **TNF α** | **IL-1 β** |
| | | **% inhibition** | **% inhibition** |
| **25** | 10 | 15.39 | 38.5 |
| | 100 | 64.06 | 79.4 |
| **46** | 10 | 40.9 | 73.3 |
| | 100 | 90.1 | 95.8 |
| **47** | 10 | 24.4 | 78.1 |
| | 100 | 89.6 | 98.5 |
| **60** | 10 | 36.66 | 35.19 |
| | 100 | 81.72 | 84.46 |
| **24** | 10 | 7.43 | 22.7 |
| | 100 | 69.72 | 81.2 |
| **43** | 10 | 17.79 | 24.62 |
| | 100 | 55.14 | 79.29 |
| **44** | 10 | 32.74 | 64.82 |
| | 100 | 95.59 | 92.36 |
| **36** | 10 | 34.31 | 38.85 |
| | 100 | 97.69 | 96.26 |

### B) Demonstration of in vivo efficacy of the compounds:

Balb/c mice were kept for acclimatization in the observation room for two days before the experiment. On the day of the experiment, animals were weighed and the test compounds to be administered calculated at a 10 mg/kg body weight basis in a total volume of 2 ml. Control groups received vehicle alone while the treatment groups received the test compound, both given orally, 30 minutes prior to intravenous injection of LPS (50µg/kg). Blood was collected 60 minutes after the LPS injection from retro orbital plexus, the serum separated & stored in deep freeze till the estimation of TNF by ELISA method.

| **Ex. No.** | **In vivo LPS induced TNF α inhibition in Balb/c mice** |
|---|---|
| | **TNF α** |
| | **% inhibition (10mg/kg)** |
| **25** | 59.6 |
| **46** | 56.80 |
| **47** | 48.60 |
| **60** | 53.65 |
| **24** | 39.3 |
| **43** | 49.4 |
| **44** | 50.4 |
| **36** | 43.3 |

### C) Demonstration of in-vitro activity

The compounds were screened using an *in vitro* ELISA assay for p38 MAP kinase activity. Activated p38 MAP kinase during the course of its biological function phosphorylates its substrates. The assay was based on the detection of a phosphorylated p38 MAP kinase substrate using ATF-2 as the biological substrate. More the activity of the p38 MAP kinase inhibitor in the reaction mixture less will be the amount of active p38 MAP kinase available to phosphorylate ATF-2. So, lower OD value will indicate the higher inhibition of p38 MAP kinase by the specific p38 MAP kinase inhibitor. The quantitation of phosphorylated ATF-2 at varying concentrations of p38 MAP kinase inhibitor was used to determine the IC₅₀ by fitting the results to a logistic dose-response program (Graphpad Prism, CA). IC₅₀ was defined as the concentration of compound required to achieve 50 % inhibition of p38 MAP kinase activity.
This can be seen in Table 3 below.

**Table 3:**

| **Example No.** | **p-38 MAPK Enzyme assay IC₅₀ (µ m)** |
|---|---|
| 4 | 0.78 |
| 2 | 1.2 |
| 3 | 1.1 |
| 4 | 1.12 |
| 5 | 1.4 |
| 6 | 0.15 |
| 7 | 3.8 |
| 8 | 55.4 |
| 9 | 5.42 |
| 10 | 11.1 |
| 11 | 2.0 |
| 12 | 6.95 |
| 13 | 1.3 |
| 14 | 6.2 |
| 15 | 4.9 |
| 16 | 1.9 |
| 17 | 10 |
| 18 | 15 |
| 19 | 6.8 |
| 20 | 0.8 |
| 21 | 2.7 |
| 22 | 5.01 |
| 23 | 6.6 |
| 24 | 9.8 |
| 27 | 3.1 |
| 28 | 0.46 |
| 29 | 2.6 |
| 31 | 3.2 |
| 32 | 10 |
| 33 | 3.9 |
| 34 | 6.4 |
| 35 | 14.6 |
| 36 | 13.4 |
| 37 | 5.93 |
| 38 | 0.17 |
| 39 | 4.6 |
| 40 | 2.7 |
| 41 | 1.4 |
| 42 | 7.1 |
| 43 | 71 |
| 44 | 6.6 |
| 45 | 1.35 |
| 50 | 4.4 |
| 53 | 1.9 |
| 54 | 4.8 |
| 57 | 6.5 |
| 58 | 1.0 |
| 59 | 6.6 |
| 60 | 11.8 |
| 64 | 9.4 |
| 70 | 8.2 |
| 71 | 25.5 |
| 72 | 3.9 |
| 74 | 7.5 |
| 75 | 12.6 |

## Claims

1. Compounds of the general formula (I) 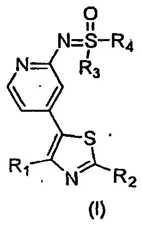 and their stereoisomers, tautomeric forms, and their pharmaceutically acceptable salts and solvates, wherein R₁ and R₂ may be the same or different and independently represent hydrogen, optionally substituted groups selected from linear or branched (C₁-C₆)alkyl; linear or branched (C₂-C₆)alkenyl, linear or branched (C₂-C₆)alkynyl, (C₃-C₇)cycloalkyl, (C₃-C₇)cycloalkenyl, aryl, heteroaryl, heterocyclyl groups, each of the cyclic groups may optionally be fused; R₃ and R₄ may be the same or different and may independently be selected from optionally substituted linear or branched (C₁-C₆)alkyl, (C₃-C₇)cycloalkyl, aryl, heteroaryl, heterocyclyl systems, each of these cyclic systems may be optionally fused, or R₃ & R₄ may, together with the sulphur atom to which they are attached, form a 3-7 membered ring system, which may optionally contain from 1-3 heteroatoms selected from N, O or S.

2. A compound as claimed in claim 1 wherein aryl group may be an aromatic system containing one two or three rings wherein such rings may be attached together in a pendant manner or may be fused, more preferably the aryl groups are selected from phenyl, naphthyl, tetrahydronaphthyl, indane, biphenyl groups.

3. A compound as claimed in claim 1 wherein the heteroaryl group represents 5 to 8 membered aromatic radicals, which may be single or fused containing one or more hetero atoms selected from O, N or S.

4. A compound as claimed in claim 1 or 3 wherein the hetetoaryl group is preferably selected from pyridyl, thienyl, furyl, pyrrolyl, oxazolyl, thiazolyl, isothiazolyl, imidazolyl, isoxazolyl, oxadiazolyl, thiadiazolyl, triazolyl, tetrazolyl, benzopyranyl, benzopyranonyl, benzofuranyl, benzothienyl, indolinyl, indolyl, azaindolyl, azaindolinyl, benzodihydrofuranyl, benzodihydrothienyl, pyrazolopyrimidinyl, pyrazotopyrimidonyl, azaquinazolinyl, azaquinazolinoyl, pyridofuranyl,
pyridothienyl, thienopyrimidyl, thienopyrimidonyl, quinolinyl, pyrimidinyl, pyrazolyl, quinazolinyl, quinazolonyl, pyrimidonyl, pyridazinyl, triazinyl, benzoxazinyl, benzoxazinonyl, benzothiazinyl, benzothiazinonyl, benzoxazolyl, benzothiazolyl, benzimidazolyl, benzotriazolyl, phthalazynil, naphthylidinyl, purinyl, carbazolyl, phenothiazinyl, phenoxazinyl groups.

5. A compound as claimed in claim 1 wherein the heterocyclyl represents saturated, partially saturated and unsaturated ring-shaped radicals, the heteroatoms selected from nitrogen, sulfur and oxygen.

6. A compound as claimed in claim 1 or 5 wherein the heterocyclyl is preferably selected from aziridinyl, azetidinyl, pyrrolidinyl, imidazolidinyl, piperidinyl, piperazinyl, 2-oxopiperidinyl, 4-oxopiperidinyl, 2-oxopiperazinyl, 3-oxopiperazinyl, morpholinyl, thiomorpholinyl, 2-oxomorpholinyl, azepinyl, diazepinyl, oxapinyl, thiazepinyl, oxazolidinyl, thiazolidinyl, and the like; examples of partially saturated heterocyclic radicals include dihydrothiophene, dihydropyran, dihydrofuran, dihydrothiazole groups.

7. A compound as claimed in any of the preceding claims wherein substituents may be selected from hydroxyl, oxo, halo, thio, nitro, amino, imino, cyano, formyl, or optionally substituted groups selected from alkyl, haloalkyl, hydroxyalkyl, aminoalkyl, thioalkyl, alkoxy, haloalkoxy, alkoxyalkyl, acyl, monosubstituted or disubstituted amino, carboxylic acid and its derivatives such as esters and amides.

8. A compound as claimed in any preceding claim preferably selected from
S-Cyclopentyl-S-phenyl-N-[4-(2-ethyl-4-(3-methylphenyl)-1,3-thiazol-5-yl)-pyridine-2-yl]-sulfoximine;
S,S-Dicyclohexyl-N-[4-(2-ethyl-4-m-tolyl-thiazol-5-yl)-pyridine-2-yl]-sulfoximine;
S-methyl-S-4-methoxyphexyl-N-[4-(2-ethyl-4-m-tolyl-thiazol-5-yl)-pyridine-2-yl]-sulfoximine;
S-Methyl-S-phenyl-N-[4-(2-ethyl-4-(3-methylphenyl)-1,3-thiazol-5-yl)-pyridine-2-yl]-sulfoximine;
(+)-(S)-Methyl-S-phenyl-N-[4-(2-ethyl-4-(3-methylphenyl)-1,3-thiazol-5-yl)-pyridine-2-yl]-sulfoximine;
(-)-(S)-Methyl-S-phenyl-N-[4-(2-ethyl-4-(3-methylphenyl)-1,3-thiazol-5-yl)-pyridine-2-yl]-sulfoximine;
S-Methyl-S-(4-fluorophenyl)-N-[4-(2-ethyl-4-(3-methylphenyl)-1,3-thiazol-5-yl)-pyridine-2-yl]-sulfoximine;
S-Isobutyl-S-phenyl-N-[4-(2-ethyl-4-m-tolyl-thiazol-5-yl)-pyridin-2-yl]-sulfoximine;
S-(3-Fluorophenyl)-S-methyl-N-[4-(2-ethyl-4-tolyl-thiazol-5-yl)-pyridin-2-yl]-sulfoximine;
S-(3-Methylphenyl)-S-methyl-N-[4-(2-ethyl-4-in-tolyl-thiazol-5-yl)-pyridin-2-yl]-sulfoximine;
S, S-Diphenyl-N-[4-(2-ethyl-4-m-tolyl-thiazol-5-yl)-pyridin-2-yl]-sulfoximine;
S-(2-Methylphenyl)-S-methyl-N-[4-(2-ethyl-4-m-tolyl-thiazol-5-yl)-pyridin-2-yl]-sulfoximine;
S-Isopropyl-S-phenyl-N-[4-(2-ethyl-4-(3-methylphenyl)-1,3-thiazol-5-yl)-pyridine-2-yl]-sulfoximine;
S-Ethyl-S-phenyl-N-[4-(2-ethyl-4-(3-methylphenyl)-1,3-thiazol-5-yl)-pyridine-2-yl]-sulfoximine;
S-Methyl-S-4-methylphenyl-N-[4-(2-ethyl-4-(3-methylphenyl)-1,3-thiazol-5-yl)-pyridine-2-yl]-sulfoximine;
Di-n-butyl-N-[4-(2-ethyl-4-(3-methylphenyl)-1,3-thiazol-5-yl)-pyridine-2-yl]-sulfoximine;
S-Methyl-S-(3-chloro-4-flurophenyl)-N-[4-(2-ethyl-4-(3-methylphenyl)-1,3-thiazol-5-yl)-pyridine-2-yl]-sulfoximine;
1-Imino-N-[4-(2-ethyl-4-(3-methylpbenyl)-1,3-thiazol-5-yl)-pyridine-2-yl]-tetrahydro-2H-thiopyran-1-oxide;
1-Imino-N-[4-(2-ethyl-4-(3-methylphenyl)-1,3-thiazol-5-yl)-pyridine-2-yl]-tetrahydro-thiophene-1-oxide;
(-)-(S)-Methyl-S-phenyl-N-[4-(2-ethyl-4phenyl)-1,3-thiazole-5-yl)-pyridine-2-yl]-sulfoximine;
(+)-S-Methyl-S-phenyl-N-[4-(2-ethyl-4phenyl)-1,3-thiazole-5-yl)-pyridine-2-yl]-sulfoximine;
S-Isopropyl-S-phenyl-N-[4-(2-ethyl-4-phenyl)-1,3-thiazole-5-yl)-pyridine-2-yl]-sulfoximine;
S-(4-Methoxyphenyl)-S-methyl-N-[4-(2-ethyl-4-phenyl)-1,3-thiazole-5-yl)-pyridine-2-yl]-sulfoximine;
S-Methyl-S-(3-methylphenyl)-N-[4-{2-ethyl-4-phenyl-thiazol-5-yl}-pyridine]-sulfoximine;
S-Methyl-S-(3-fluorophenyl)-N-[4-{2-ethyl-4-phenyl-thiazol-5-yl}-pyridine]-sulfoximine;
4-[2-Ethyl-4-phenyl-thiazol-5-yl]-2-(dicyclohexyl sulfoxitnine)-pyridine;
S-Methyl-S-(4-fluorophenyl-N-[4-(2-ethyl-4-phenyl-thiazol-5-yl)pyridine]-sulfoximine;
S-Cyclopentyl-S-phenyl-N-[4-{2-ethyl-4-phenyl-thiazol-5-yl}-pyridine]-sulfoximine;
(-)-S-Cyclo-pentyl-S-phenyl-N-[4-(2-ethyl-4-phenyl)-1,3-thiazol-5-yl)-pyridine-2-yl]-sulfoximine;
(+)-S-Cyclopentyl-S-phenyl-N-[4-(2-ethyl-4-phenyl)-1,3-thiazol-5-yl)-pyridine-2-yl]-sulfoximine;
S-Methyl-S-phenyl-N-[-4-(2-ethyl-4-phenyl)-1,3-thiazol-5-yl)-pyridine-2-yl]-sulfoximine;
S,S-Diphenyl-N-[4-(2-ethyl-4-phenyl-thiazol-5-yl)-pyridin-2-yl]-sulfoximine; (-)-S-Methyl-S-phenyl-N-{4-[2-ethyl-4-(4-fluoro-phenyl)-thiazol-5-yl]-pyridin-2-yl}-
sulfoximine;
S-(4-Methoxy-phenyl)-S-methyl-N-{4-[2-ethyl-4-(4-fluoro-phenyl)-thiazol-5-yl]-pyridin-2-yl}-sulfoximine;
S-Isopropyl-S-phenyl-N-{4-[2-ethyl-4-(4-fluoro-phenyl)-thiazol-5-yl]-pyridin-2-yl}-sulfoximine;
(-)-Isopropyl-S-phenyl-N-{4-[2-ethyl-4-(4-fluoxo-phenyl)-thiazol-5-yl]-pyridin-2-yl}-sulfoximine;
(+)-S-isopropyl-S-phenyl-N-{4-[2-ethyl-4-(4-fluoro-phenyl)-thiazol-5-yl]-pyridin-2-yl}-sulfoximine;
S-Ethyl-S-phenyl-N-{4-[2-ethyl-4-(4-fluoro-phenyl)-thiazol-5-yl]-pyridin-2-yl}-sulfoximine;
S-Methyl-S-phenyl-N-[4-(2-ethyl-4-(4-fluoro-phenyl)-1,3-thiazol-5-yl)-pyridine-2-yl]-sulfoximine;
(+)-S-Methyl-S-phenyl-N-[4-(2-ethyl-4-(4-fluoro-phenyl)-1,3-thiazol-5-yl)-pyridine-2-yl]-sulfoximine;
S-Methyl-S-4-fluoro-phenyl-N-[4-(2-ethyl-4-(4-fluoro-phenyl)-1,3-thiazol-5-yl)-pyridine-2-yl]-sulfoximine;
S-Cyclopentyl-S-phenyl-N-[4-(2-ethyl-4-(4-fluorophenyl)-1,3-thiazol-5-yl)-pyridine-2-yl]-sulfoximine;
(-)-S-Methyl-S-4-fluoro-phenyl-N-[4-(2-ethyl-4-(4-fluoro-phenyl)-1,3-thiazol-5-yl)-pyridine-2-yl]-sulfoximine;
(+)-S-Methyl-S-4-fluoro-phenyl-N-[4-(2-ethyl-4-(4-fluoro-phenyl)-1,3-thiazol-5-yl)-pyridine-2-yl]-sulfoximine;
S,S-Diphenyl-N-[4-(2-ethyl-4-(4-fluoro phenyl)-thiazol-5-yl)-pyridin-2-yl]-sulfoximine;
S-Met-hyl-S-3-methylphenyl-N-[4-(2-ethyl-4-fluorophenyl-thiazol-5-yl)-pyridine]-sulfoximine;
S-Methyl-S-3-fluorophenyl-N-[4-{2-ethyl-4-(4-fluorophenyl)-thiazol-5-yl}-pyridine-2yl]-sulfoximine;
S-Cyclohexyl-S-phenyl-N-{4-[2-ethyl-4-(4-fluorophenyl)-thiazol-5-yl]-pyridine}-sulfoximine;
S-Methyl-S-phenyl-N-[4-(2-ethyl-4-m-benzoic acid-thiazol-5-yl)-pyridine-2-yl]-sulfoximine;
S-Methyl-S-phenyl-N-[4-(2-ethyl-4-(3-fluoro-phenyl)-1,3-thiazol-5-yl)-pyridine-2-yl]-sulfoximine;
S-lsopropyl-S-phenyl-N-[4-(2-ethyl-4-(3-fluoro-phenyl)-1,3-thiazol-5-yl)-pyridine-2-yl]-sulfoximine;
S-Cyclopentyl-S-phenyl-N-[4-(2-ethyl-4-(3-fluoro-phenyl)-1,3-thiazol-5-yl)-pyridine-2-yl]-sulfoximine;
S-Methyl-S-phenyl-N-{4-[2-ethyl-4-naphthalene-1-yl-thiazol-5-yl]-pyridin-2-yl}-sulfoximine;
S-Methyl-S-phenyl-N-{4-[2-ethyl-4-(3-trifluoromethylphenyl)-thiazol-5-yl]-pyridin-2-yl}-sulfoximine;
S-Methyl-S-phenyl-N-{4-[2-ethyl-4-(4-methoxyphenyl)-thiazol-5-yl]-pyridin-2-yl}-sulfoximine;
S-Methyl-S-phenyl-N-{4-[2-(4-methylsulfanyl-phenyl)-4-m-tolyl-thiazol-5-yl]-pyridin-2-yl}-sulfoximine;
S-Methyl-S-phenyl-N-{4-[2-(4-methylsulfinyl-phenyl)-4-m-tolyl-thiazol-5-yl]-pyridin-2-yl}-sulfoximine;
S-Methyl-S-phenyl-N-{4-[2-ethyl-4-(3,4-difluorophenyl)-thiazol-5-yl]-pyridin-2-yl}-sulfoximine;
S-Methyl-(4-fluorophenyl)-N-{4-[2-ethyl-4-(3,4-difluorophenyl)-thiazol-5-yl]-pyridin-2-yl}-sulfoximine;
S-isopropyl-S-phenyl-N-{4-[2-ethyl-4-(3,4-difluorophenyl)-thiazol-5-yl]-pyridin-2-yl}-sulfoximine;
S-isopropyl-S-phenyl-N-{4-[2-ethyl-4-(3,5-difluorophenyl)-thiazol-5-yl]-pyridin-2-yl}-sulfoximine;
S-Methyl-S-phehyl-N-{4-[2-ethyl-4-(3,5-difluorophenyl)-thiazol-5-yl]-pyridin-2-yl}-sulfoximine;
S,S-Diphenyl-N-{4-[2-ethyl-4-(3,5-difluorophenyl)-thiazol-5-yl]-pyridin-2-yl}-sulfoximine;
S-Methyl-S-phenyl-N-{4-[2-ethyl-4-p-tolyl -thiazol-5-yl]-pyridin-2-yl}-sulfoximine;
S-Cyclopentyl-S-phenyl-N-{4-[2-(2,6-difluorophenyl)-4-(3-fluorophenyl)-(1,3)-thiazol-5-yl]-pyridin-2-yl}-sulfoximine;
S-Isopropyl-S-phenyl-N-{4-[2-(2,6-difluorophenyl)-4-(3-fluorophenyl)-(1,3)-thiazol-5-yl]-pyridin-2-yl}-sulfoximine;
S-Cyclohexyl-S-phenyl-N-{4-[2-(2,6-difluorophenyl)-4-(3-fluorophenyl)-(1,3)-thiazol-5-yl]-pyridin-2-yl}-sulfoximine;
S-Cyclohexyl-S-phenyl-N-{4-[2-(4-fluorophenyl)-4-(3-fluorophenyl)-(1,3)-thiazol-S-yl]-pyridin-2-yl}-sulfoximine;
S-Isopropyl-S-phenyl-N-{4-[2-(4-fluorophenyl)-4-(3-fluorophenyl)-(1,3)-thiazol-5-yl]-pyridin-2-yl}-sulfoximine;
Methane sulfonate salt of (+)-S-Isopropyl-S-phenyl-N-{4-[2-ethyl-4-(4-fluorophenyl)-(1,3)-thiazol-5-yl]-pyridin-2-yl}-sulfoximine;
N-Oxide of (+)-S-isopropyl,S-phenyl-N-{4-[2-ethyl-4-(4-fluorophenyl)-(1,3)-thiazol-5-yl]-pyridin-2-yl}-sulfoximine methane sulfonate salt;
S-Cyclopentyl-S-phenyl-N-[4-(3-fluoropheriyl)-1,3-thiazol-5-yl)-pyridine-2-yl]-sulfoximine;
S-Methyl-S-phenyl-N-[4-(3-fluorophenyl)-1,3-thiazol-5-yl)-pyridine-2-yl]-sulfoximine;
S-Isopropyl-S-phenyl-N-[4-(3-fluorophenyl)-1,3-thiazol-S-yl)-pyridine-2-yl]-sulfoximine;
S-Methyl-S-phenyl-N-[4-(4-fluorophenyl)-1,3-thiazol-5-yl-pyridine-2-yl]-sulfoximine;
S-Isopropyl-S-phenyl-N-[4-(4-fluorophenyl)-1,3-thiazol-5-yl)-pyridine-2-yl]-sulfoximine;
S-Cyclopentyl-S-phenyl-N-[4-(4-fluorophenyl)-1,3-thiazol-5-yl)-pyridine-2-yl]-sulfoximine;
S-Methyl-S-phenyl-N-[4-phenyl-(1,3)-thiazol-5-yl-pyridine-2-yl]-sulfoximine;
S-lsopropyl-S-phenyl-N-[4-phenyl-1,3-thliazol-5-yl)-pyridine-2-yl]-sulfoximine;
S-Cyclopentyl-S-phenyl-N-[(4-phenyl-thiazol-5-yl)-pyridina-2-yl]- sulfoximine;

9. A pharmaceutical composition which comprises compounds of formula (I), as claimed in any preceding claims and a pharmaceutically acceptable carrier, diluent or excipients.

10. A process for-preparing compound of formula (I) as claimed in any preceding claims comprising the steps of reacting a compound of formula (II) wherein 'X' represents suitable leaving group with sulfoximine compound of formula (III) using suitable coupling catalyst(s) selected from palladium acetate, copper salts in the presence of suitable ligand(s) selected from N,N'-dimethylethyl diamine (DMEDA), and in the presence of suitable inorganic base(s) selected from cesium carbonate, cesium acetate, potassium carbonate, potassium phosphate, potassium hydroxide, sodium hydroxide, sodium carbonate, lithium hydroxide, sodium hydride, potassium hydride or mixtures thereof to yield a compound of formula (I), wherein each of the terms are as defined in the specification

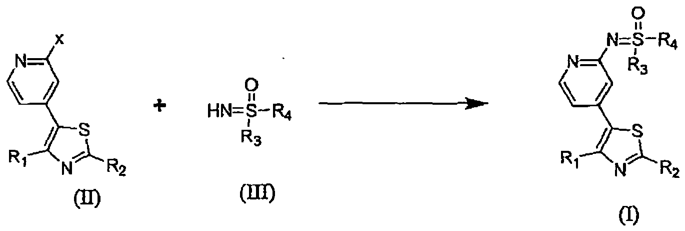

11. A compound as defined in any one of claims 1 to 8, or a composition as defined in claim 9, for use in the treatment of the human or animal body by therapy.

12. A compound as defined in any one of claims 1 to 8, or a composition as defined in claim 9, for use in a method of treating rheumatoid arthritis (RA).

13. Use of a compound as defined in any one of claims 1 to 8, or a composition as defined
in claim 9, in the manufacture of a medicament for use in the treatment of rheumatoid arthritis (RA).

## Patentansprüche

1. Verbindungen der allgemeinen Formel (I) 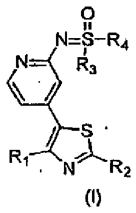 und deren Stereoisomere, tautomere Formen, und deren pharmazeutisch akzeptable Salze und Solvate, worin R₁ und R₂ gleich oder verschieden sein können und unabhängig voneinander für Wasserstoff, optional substituierte Gruppen, ausgewählt aus linearem oder verzweigtem (C₁-C₆)-Alkyl, linearem oder verzweigtem (C₂-C₆)-Alkenyl, linearem oder verzweigtem (C₂-C₆)-Alkynyl, (C₃-C₇)-Cycloalkyl, (C₃-C₇)-Cycloalkenyl, Aryl, Heteroaryl, Heterocyclyl-Gruppen, wobei jede der cyclischen Gruppen optional fusioniert sein kann, stehen; R₃ und R₄ gleich oder verschieden sein können und unabhängig voneinander ausgewählt sein können aus optional substituiertem linearem oder verzweigtem (C₁-C₆)-Alkyl, (C₃-C₇)-Cycloalkyl, Aryl, Heteroaryl, Heterocyclyl-Systemen, wobei jedes dieser cyclischen Systeme optional fusioniert sein kann, oder R3 und R4, zusammen mit dem Schwefelatom, an das sie gebunden sind, ein 3-7-gliedriges Ringsystem bilden können, welches optional 1-3 Heteroatome enthalten kann, ausgewählt aus N, O oder S.

2. Verbindung nach Anspruch 1, worin die Arylgruppe ein aromatisches System sein kann, enthaltend ein, zwei oder drei Ringe, wobei diese Ringe in einer hängenden Weise aneinander gebunden sein können oder fusioniert sein können, und bevorzugter die Arylgruppen ausgewählt sind aus Phenyl-, Naphthyl-, Tetrahydronaphthyl-, Indan-, Biphenylgruppen.

3. Verbindung nach Anspruch 1, worin die Heteroarylgruppe für 5- bis 8-gliedrige aromatische Radikale steht, die einzeln oder fusioniert sein können, enthaltend ein oder mehrere Heteroatome, ausgewählt aus O, N oder S.

4. Verbindung nach Anspruch 1 oder 3, worin die Heteroarylgruppe vorzugsweise ausgewählt ist aus Pyridyl-, Thienyl-, Furyl-, Pyrrolyl-, Oxazolyl-, Thiazolyl-, Isothiazolyl-, Imidazolyl-, Isoxazolyl-, Oxadiazolyl-, Thiadiazolyl-, Triazolyl-, Tetrazolyl-, Benzopyranyl-, Benzopyranonyl-, Benzofuranyl-, Benzothienyl-, Indolinyl-, Indolyl-, Azaindolyl-, Azaindolinyl-, Benzodihydrofuranyl-, Benzodihydrothienyl-, Pyrazolopyrimidinyl-, Pyrazolopyrimidonyl-, Azachinazolinyl-, Azachinazolinoyl-, Pyridofuranyl-, Pyridothienyl-, Thienopyrimidyl-, Thienopyrimidonyl-, Chinolinyl-, Pyrimidinyl-, Pyrazolyl-, Chinazolinyl-, Chinazolonyl-, Pyrimidonyl-, Pyridazinyl-, Triazinyl-, Benzoxazinyl-, Benzoxazinonyl-, Benzothiazinyl-, Benzothiazinonyl-, Benzoxazolyl-, Benzothiazolyl-, Benzimidazolyl-, Benzotriazolyl-, Phthalazynil-, Naphthylidinyl-, Purinyl-, Carbazolyl-, Phenothiazinyl-, Phenoxazinylgruppen.

5. Verbindung nach Anspruch 1, worin das Heterocyclyl für gesättigte, teilweise gesättigte und ungesättigte ringförmige Radikale steht, wobei die Heteroatome ausgewählt sind aus Stickstoff, Schwefel und Sauerstoff.

6. Verbindung nach Anspruch 1 oder 5, worin das Heterocyclyl vorzugsweise ausgewählt ist aus Aziridinyl, Azetidinyl, Pyrrolidinyl, Imidazolidinyl, Piperidinyl, Piperazinyl, 2-Oxopiperidinyl, 4-Oxopiperidinyl, 2-Oxopiperazinyl, 3-Oxopiperazinyl, Morpholinyl, Thiomorpholinyl, 2-Oxomorpholinyl, Azepinyl, Diazepinyl, Oxapinyl, Thiazepinyl, Oxazolidinyl, Thiazolidinyl und ähnlichem; Beispiele der teilweise gesättigten heterocyclischen Radikale umfassen Dihydrothiophen-, Dihydropyran-, Dihydrofuran-, Dihydrothiazolgruppen.

7. Verbindung nach jedem der vorangehenden Ansprüche, worin Substituenten ausgewählt sein können aus Hydroxyl, Oxo, Halo, Thio, Nitro, Amino, Imino, Cyano, Formyl, oder optional substituierten Gruppen, ausgewählt aus Alkyl, Halogenalkyl, Hydroxyalkyl, Aminoalkyl, Thioalkyl, Alkoxy, Halogenalkoxy, Alkoxyalkyl, Acyl, monosubstituiertem oder disubstituiertem Amino, Carbonsäure und ihren Derivaten, wie etwa Estern und Amiden.

8. Verbindung nach jedem der vorangehenden Ansprüche, vorzugsweise ausgewählt aus:
S-Cyclopentyl-S-phenyl-N-[4-(2-ethyl-4-(3-methylphenyl)-1,3-thiazol-5-yl)-pyridin-2-yl]-sulfoximin;
S,S-Dicyclohexyl-N-[4-(2-ethyl-4-m-tolylthiazol-5-yl)-pyridin-2-yl]-sulfoximin;
S-Methyl-S-4-methoxyphenyl-N-[4-(2-ethyl-4-m-tolylthiazol-5-yl)-pyridin-2-yl]-sulfoximin;
S-Methyl-S-phenyl-N-[4-(2-ethyl-4-(3-methylphenyl)-1,3-thiazol-5-yl)-pyridin-2-yl]-sulfoximin;
(+)-S-Methyl-S-phenyl-N-[4-(2-ethyl-4-(3-methylphenyl)-1,3-thiazol-5-yl)-pyridin-2-yl]-sulfoximin;
(-)-S-Methyl-S-phenyl-N-[4-(2-ethyl-4-(3-methylphenyl)-1,3-thiazol-5-yl)-pyridin-2-yl]-sulfoximin;
S-Methyl-S-(4-fluorphenyl)-N-[4-(2-ethyl-4-(3-methylphenyl)-1,3-thiazol-5-yl)-pyridin-2-yl]-sulfoximin;
S-Isobutyl-S-phenyl-N-[4-(2-ethyl-4-m-tolylthiazol-5-yl)-pyridin-2-yl]-sulfoximin;
S-(3-Fluorphenyl)-S-methyl-N-[4-(2-ethyl-4-m-tolylthiazol-5-yl)-pyridin-2-yl]-sulfoximin;
S-(3-Methylphenyl)-S-methyl-N-[4-(2-ethyl-4-m-tolyl-thiazol-5-yl)-pyridin-2-yl]-sulfoximin;
S,S-Diphenyl)-N-[4-(2-ethyl-4-m-tolyl-thiazol-5-yl)-pyridin-2-yl]-sulfoximin;
S-(2-Methylphenyl)-S-methyl-N-[4-(2-ethyl-4-m-tolyl-thiazol-5-yl)-pyridin-2-yl]-sulfoximin;
S-Isopropyl-S-phenyl-N-[4-(2-ethyl-4-(3-methylphenyl)-1,3-thiazol-5-yl)-pyridin-2-yl]-sulfoximin;
S-Ethyl-S-phenyl-N-[4-(2-ethyl-4-(3-methylphenyl)-1,3-thiazol-5-yl)-pyridin-2-yl]-sulfoximin;
S-Methyl-S-4-methylphenyl-N-[4-(2-ethyl-4-(3-methylphenyl)-1,3-thiazol-5-yl)-pyridin-2-yl]-sulfoximin;
Di-n-butyl-N-[4-(2-ethyl-4-(3-methylphenyl)-1,3-thiazol-5-yl)-pyridin-2-yl]-sulfoximin;
S-Methyl-S-(3-chlor-4-fluorphenyl)-N-[4-(2-ethyl-4-(3-methylphenyl)-1,3-thiazol-5-yl)-pyridin-2-yl]-sulfoximin;
1-Imino-N-[4-(2-ethyl-4-(3-methylphenyl)-1,3-thiazol-5-yl)-pyridin-2-yl]-tetrahydro-2H-thiopyran-1-oxid;
1-Imino-N-[4-(2-ethyl-4-(3-methylphenyl)-1,3-thiazol-5-yl)-pyridin-2-yl]-tetrahydrothiophen-1-oxid;
(-)-S-Methyl-S-phenyl-N-[4-(2-ethyl-4-phenyl)-1,3-thiazol-5-yl)-pyridin-2-yl]-sulfoximin;
(+)-S-Methyl-S-phenyl-N-[4-(2-ethyl-4-phenyl)-1,3-thiazol-5-yl)-pyridin-2-yl]-sulfoximin;
S-Isopropyl-S-phenyl-N-[4-(2-ethyl-4-phenyl)-1,3-thiazol-5-yl)-pyridin-2-yl]-sulfoximin;
S-(4-Methoxyphenyl)-S-methyl-N-[4-(2-ethyl-4-phenyl)-1,3-thiazol-5-yl)-pyridin-2-yl]-sulfoximin;
S-Methyl-S-(3-methylphenyl)-N-[4-(2-ethyl-4-phenylthiazol-5-yl)-pyridin]-sulfoximin;
S-Methyl-S-(3-fluorphenyl)-N-[4-(2-ethyl-4-phenylthiazol-5-yl)-pyridin]-sulfoximin;
4-[2-Ethyl-4-phenylthiazol-5-yl]-2-(dicyclohexylsulfoximin)-pyridin;
S-Methyl-S-(4-fluorphenyl-N-[4-(2-ethyl-4-phenylthiazol-5-yl)-pyridin]-sulfoximin;
S-Cyclopentyl-S-phenyl-N-[4-(2-ethyl-4-phenylthiazol-5-yl)-pyridin]-sulfoxim in;
(-)-S-Cyclopentyl-S-phenyl-N-[4-(2-ethyl-4-phenyl)-1,3-thiazol-5-yl)-pyridin-2-yl]-sulfoximin;
(+)-S-Cyclopentyl-S-phenyl-N-[4-(2-ethyl-4-phenyl)-1,3-thiazol-5-yl)-pyridin-2-yl]-sulfoximin;
S-Methyl-S-phenyl-N-[4-(2-ethyl-4-phenyl)-1,3-thiazol-5-yl)-pyridin-2-yl]-sulfoximin;
S,S-Diphenyl-N-[4-(2-ethyl-4-phenylthiazol-5-yl)-pyridin-2-yl]-sulfoximin;
(-)-S-Methyl-S-phenyl-N-{4-[2-ethyl-4-(4-fluorphenyl)-thiazol-5-yl]-pyridin-2-yl}-sulfoximin;
S-(4-Methoxyphenyl)-S-methyl-N-{4-[2-ethyl-4-(4-fluorphenyl)-thiazol-5-yl]-pyridin-2-yl}-sulfoximin;
S-Isopropyl-S-phenyl-N-{4-[2-ethyl-4-(4-fluorphenyl)-thiazol-5-yl]-pyridin-2-yl}-sulfoximin;
(-)-Isopropyl-S-phenyl-N-{4-[2-ethyl-4-(4-fluorphenyl)-thiazol-5-yl]-pyridin-2-yl}-sulfoximin;
(+)-S-Isopropyl-S-phenyl-N-{4-[2-ethyl-4-(4-fluorphenyl)-thiazol-5-yl]-pyridin-2-yl}-sulfoximin;
S-Ethyl-S-phenyl-N-{4-[2-ethyl-4-(4-fluorphenyl)-thiazol-5-yl]-pyridin-2-yl}-sulfoximin;
S-Methyl-S-phenyl-N-{4-[2-ethyl-4-(4-fluorphenyl)-1,3-thiazol-5-yl]-pyridin-2-yl}-sulfoximin;
(+)-S-Methyl-S-phenyl-N-{4-[2-ethyl-4-(4-fluorphenyl)-1,3-thiazol-5-yl]-pyridin-2-yl}-sulfoximin;
S-Methyl-S-4-fluorphenyl-N-{4-[2-ethyl-4-(4-fluorphenyl)-1,3-thiazol-5-yl]-pyridin-2-yl}-sulfoximin;
S-Cyclopentyl-S-phenyl-N-{4-[2-ethyl-4-(4-fluorphenyl)-1,3-thiazol-5-yl]-pyridin-2-yl}-sulfoximin;
(-)-S-Methyl-S-4-fluorphenyl-N-{4-[2-ethyl-4-(4-fluorphenyl)-1,3-thiazol-5-yl]-pyridin-2-yl}-sulfoximin;
(+)-S-Methyl-S-4-fluorphenyl-N-{4-[2-ethyl-4-(4-fluorphenyl)-1,3-thiazol-5-yl]-pyridin-2-yl}-sulfoximin;
S,S-Diphenyl-N-{4-[2-ethyl-4-(4-fluorphenyl)-thiazol-5-yl]-pyridin-2-yl}-sulfoximin;
S-Methyl-S-3-methylphenyl-N-[4-(2-ethyl-4-fluorphenylthiazol-5-yl)-pyridin]-sulfoximin;
S-Methyl-S-3-fluorphenyl-N-{4-[2-ethyl-4-(4-fluorphenyl)-thiazol-5-yl]-pyridin-2-yl}-sulfoximin;
S-Cyclohexyl-S-phenyl-N-{4-[2-ethyl-4-(4-fluorphenyl)-thiazol-5-yl]-pyridin}-sulfoximin;
S-Methyl-S-phenyl-N-[4-(2-ethyl-4-m-benzoesäure-thiazol-5-yl)-pyridin-2-yl]-sulfoximin;
S-Methyl-S-phenyl-N-[4-(2-ethyl-4-(3-fluorphenyl)-1,3-thiazol-5-yl)-pyridin-2-yl]-sulfoximin;
S-Isopropyl-S-phenyl-N-[4-(2-ethyl-4-(3-fluorphenyl)-1,3-thiazol-5-yl)-pyridin-2-yl]-sulfoximin;
S-Cyclopentyl-S-phenyl-N-{4-[2-ethyl-4-(3-fluorphenyl)-1,3-thiazol-5-yl]-pyridin-2-yl}-sulfoximin;
S-Methyl-S-phenyl-N-[4-(2-ethyl-4-naphthalen-1-yl-thiazol-5-yl)-pyridin-2-yl]-sulfoximin;
S-Methyl-S-phenyl-N-{4-[2-ethyl-4-(3-trifluormethylphenyl)-thiazol-5-yl]-pyridin-2-yl}-sulfoximin;
S-Methyl-S-phenyl-N-{4-[2-ethyl-4-(4-methoxyphenyl)-thiazol-5-yl]-pyridin-2-yl}-sulfoximin;
S-Methyl-S-phenyl-N-{4-[2-(4-methylsulfanylphenyl)-4-m-tolylthiazol-5-yl]-pyridin-2-yl}-sulfoximin;
S-Methyl-S-phenyl-N-{4-[2-(4-methylsulfinylphenyl)-4-m-tolylthiazol-5-yl]-pyridin-2-yl}-sulfoximin;
S-Methyl-S-phenyl-N-{4-[2-ethyl-4-(3,4-difluorphenyl)-thiazol-5-yl]-pyridin-2-yl}-sulfoximin;
S-Methyl-(4-flourphenyl)-N-{4-[2-ethyl-4-(3,4-difluorphenyl)-thiazol-5-yl]-pyridin-2-yl}-sulfoximin;
S-Isopropyl-S-phenyl-N-{4-[2-ethyl-4-(3,4-difluorphenyl)-thiazol-5-yl]-pyridin-2-yl}-sulfoximin;
S-Isopropyl-S-phenyl-N-{4-[2-ethyl-4-(3,5-difluorphenyl)-thiazol-5-yl]-pyridin-2-yl}-sulfoximin;
S-Methyl-S-phenyl-N-{4-[2-ethyl-4-(3,5-difluorphenyl)-thiazol-5-yl]-pyridin-2-yl}-sulfoximin;
S,S-Diphenyl-N-{4-[2-ethyl-4-(3,5-difluorphenyl)-thiazol-5-yl]-pyridin-2-yl}-sulfoximin;
S-Methyl-S-phenyl-N-{4-[2-ethyl-4-p-tolylthiazol-5-yl]-pyridin-2-yl}-sulfoximin;
S-Cyclopentyl-S-phenyl-N-{4-[2-(2,6-difluorphenyl)-4-(3-fluorphenyl)-(1,3)-thiazol-5-yl]-pyridin-2-yl}-sulfoximin;
S-Isopropyl-S-phenyl-N-{4-[2-(2,6-difluorphenyl)-4-(3-fluorphenyl)-(1,3)-thiazol-5-yl]-pyridin-2-yl}-sulfoximin;
S-Cyclohexyl-S-phenyl-N-{4-[2-(2,6-difluorphenyl)-4-(3-fluorphenyl)-(1,3)-thiazol-5-yl]-pyridin-2-yl}-sulfoximin;
S-Cyclohexyl-S-phenyl-N-{4-[2-(4-fluorphenyl)-4-(3-fluorphenyl)-(1,3)-thiazol-5-yl]-pyridin-2-yl}-sulfoximin;
S-Isopropyl-S-phenyl-N-{4-[2-(4-fluorphenyl)-4-(3-fluorphenyl)-(1,3)-thiazol-5-yl]-pyridin-2-yl}-sulfoximin;
Methansulfonatsalz von (+)-S-Isopropyl-S-phenyl-N-{4-[2-ethyl-4-(4-fluorphenyl)-(1,3)-thiazol-5-yl]-pyridin-2-yl}-sulfoximin;
N-Oxid von (+)-S-Isopropyl-S-phenyl-N-{4-[2-ethyl-4-(4-fluorphenyl)-(1,3)-thiazol-5-yl]-pyridin-2-yl}-sulfoximin-Methansulfonatsalz;
S-Cyclopentyl-S-phenyl-N-[4-(3-fluorphenyl)-1,3-thiazol-5-yl-pyridin-2-yl]-sulfoximin;
S-Methyl-S-phenyl-N-[4-(3-fluorphenyl)-1,3-thiazol-5-yl-pyridin-2-yl]-sulfoximin;
S-Isopropyl-S-phenyl-N-[4-(3-fluorphenyl)-1,3-thiazol-5-yl-pyridin-2-yl]-sulfoximin;
S-Methyl-S-phenyl-N-[4-(4-fluorphenyl)-1,3-thiazol-5-yl-pyridin-2-yl]-sulfoximin;
S-Isopropyl-S-phenyl-N-[4-(4-fluorphenyl)-1,3-thiazol-5-yl-pyridin-2-yl]-sulfoximin;
S-Cyclopentyl-S-phenyl-N-[4-(4-fluorphenyl)-1,3-thiazol-5-yl-pyridin-2-yl]-sulfoximin;
S-Methyl-S-phenyl-N-[4-phenyl-(1,3)-thiazol-5-yl-pyridin-2-yl]-sulfoximin;
S-Isopropyl-S-phenyl-N-[(4-phenyl-1,3-thiazol-5-yl)-pyridin-2-yl]-sulfoximin;
S-Cyclopentyl-S-phenyl-N-[(4-phenylthiazol-5-yl)-pyridin-2-yl]-sulfoximin.

9. Pharmazeutische Zusammensetzung, welche Verbindungen der Formel (I) umfasst, wie in jedem der vorangehenden Ansprüche beansprucht, und einen pharmazeutisch akzeptablen Träger, Verdünnungsmittel oder Exzipienten.

10. Verfahren zur Herstellung der Verbindung der Formel (I), wie in jedem der vorangehenden Ansprüche beansprucht, umfassend die Schritte des Umsetzens einer Verbindung der Formel (II), worin ,X' für eine geeignete Abgangsgruppe steht, mit einer Sulfoximin-Verbindung der Formel (III), unter Verwendung eines oder mehrerer geeigneter Kopplungskatalysatoren, augewählt aus Palladiumacetat, Kupfersalzen, in der Gegenwart eines oder mehrerer geeigneter Liganden, ausgewählt aus N,N'-Dimethylethyldiamin (DMEDA), und in der Gegenwart eines oder mehrerer geeigneter anorganischer Basen, ausgewählt aus Cäsiumcarbonat, Cäsiumacetat, Kaliumcarbonat, Kaliumphosphat, Kaliumhydroxid, Natriumhydroxid, Natriumcarbonat, Lithiumhydroxid, Natriumhydrid, Kaliumhydrid oder Gemischen davon, um eine Verbindung der Formel (I) zu erhalten, worin jede der Bezeichnungen wie in der Spezifikation definiert ist:

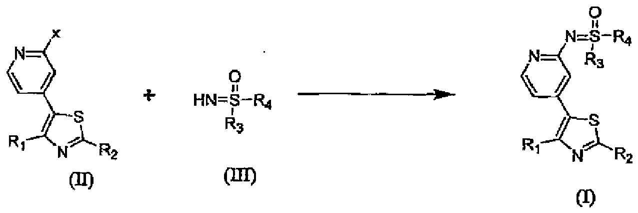

11. Verbindung, wie in jedem der Ansprüche 1 bis 8 definiert, oder Zusammensetzung, wie in Anspruch 9 definiert, zur Verwendung in der Behandlung des menschlichen oder tierischen Körpers mittels einer Therapie.

12. Verbindung, wie in jedem der Ansprüche 1 bis 8 definiert, oder Zusammensetzung, wie in Anspruch 9 definiert, zur Verwendung bei einem Verfahren zur Behandlung von rheumatoider Arthritis (RA).

13. Verwendung einer Verbindung, wie in jedem der Ansprüche 1 bis 8 definiert, oder einer Zusammensetzung, wie in Anspruch 9 definiert, in der Herstellung eines Medikaments zur Verwendung in der Behandlung einer rheumatoiden Arthritis (RA).

## Revendications

1. Composés de formule générale (I) : 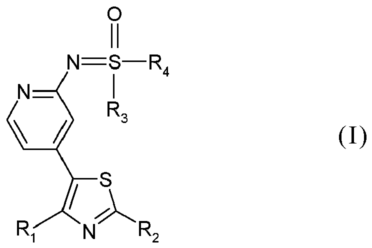 ainsi que leurs stéréoisomères, leurs formes tautomères et leurs sels et solvats pharmacologiquement admissibles, dans laquelle formule :
- R₁ et R₂ représentent des entités qui peuvent être identiques ou différentes et représentent chacun, indépendamment, un atome d'hydrogène ou un groupe, en option porteur d'un ou de substituant(s), choisi parmi les groupes alkyle en C₁₋₆ à chaîne linéaire ou ramifiée, alcényle en C₂₋₆ à chaîne linéaire ou ramifiée, alcynyle en C₂₋₆ à chaîne linéaire ou ramifiée, cycloalkyle en C₃₋₇, cycloalcényle en C₃₋₇, aryle, hétéroaryle et hétérocyclyle, étant entendu que chacun des groupes cycliques peut, en option, être un système condensé ;
- et R₃ et R₄ représentent des entités qui peuvent être identiques ou différentes et qui sont choisies indépendamment parmi les groupes, en option porteurs d'un ou de substituant(s), de type alkyle en C₁₋₆ à chaîne linéaire ou ramifiée, cycloalkyle en C₃₋₇, aryle, hétéroaryle ou hétérocyclyle, étant entendu que chacun de ces groupes cycliques peut, en option, être un système condensé, ou bien R₃ et R₄ représentent des entités qui, conjointement avec l'atome de soufre auquel elles sont liées, forment un système cyclique de 3 à 7 chaînons qui peut, en option, comporter 1 à 3 hétéroatomes choisi(s) parmi les atomes d'azote, d'oxygène et de soufre.

2. Composé conforme à la revendication 1, dans lequel un groupe aryle peut être un système aromatique comportant un cycle ou deux ou trois cycles qui peuvent être raccordés l'un à l'autre par liaison pendante ou être condensés, les groupes aryle étant de préférence choisis parmi les groupes phényle, naphtyle, tétrahydronaphtyle, indanyle et biphénylyle.

3. Composé conforme à la revendication 1, dans lequel un groupe hétéroaryle est un groupe aromatique qui comporte de 5 à 8 chaînons, qui peut être monocyclique ou à cycles condensés, et qui comporte un ou plusieurs hétéroatomes choisi(s) parmi les atomes d'azote, d'oxygène et de soufre.

4. Composé conforme à la revendication 1 ou 3, dans lequel le ou les groupe(s) hétéroaryle est ou sont de préférence choisi(s) parmi les groupes pyridyle, thiényle, furyle, pyrrolyle, oxazolyle, thiazolyle, isothiazolyle, imidazolyle, isoxazolyle, oxadiazolyle, thiadiazolyle, triazolyle, tétrazolyle, benzopyranyle, benzopyranonyle, benzofuranyle, benzothiényle, indolinyle, indolyle, azaindolyle, azaindolinyle, benzodihydrofuranyle, benzodihydrothiényle, pyrazolo-pyrimidinyle, pyrazolo-pyrimidonyle, azaquinazolinyle, azaquinazolinoyle, pyridofuranyle, pyrido-thiényle, thiéno-pyrimidyle, thiéno-pyrimidonyle, quinolyle, pyrimidinyle, pyrazolyle, quinazolinyle, quinazolonyle, pyrimidonyle, pyridazinyle, triazinyle, benzoxazinyle, benzoxazinonyle, benzothiazinyle, benzothiazinonyle, benzoxazolyle, benzothiazolyle, benzimidazolyle, benzotriazolyle, phtalazinyle, naphtylidinyle, purinyle, carbazolyle, phénothiazinyle et phénoxazinyle.

5. Composé conforme à la revendication 1, dans lequel un groupe hétérocyclyle est un groupe cyclique saturé, partiellement saturé ou insaturé, qui comporte un ou des hétéroatomes choisi(s) parmi les atomes d'azote, d'oxygène et de soufre.

6. Composé conforme à la revendication 1 ou 5, dans lequel le ou les groupe(s) hétérocyclyle est ou sont de préférence choisi(s) parmi les groupes aziridinyle, azétidinyle, pyrrolidinyle, imidazolidinyle, pipéridinyle, pipérazinyle, 2-oxo-pipéridinyle, 4-oxo-pipéridinyle, 2-oxo-pipérazinyle, 3-oxo-pipérazinyle, morpholinyle, thiomorpholinyle, 2-oxo-morpholinyle, azépinyle, diazépinyle, oxapinyle, thiazépinyle, oxazolidinyle, thiazolidinyle et autres semblables ; des exemples de groupes hétérocycliques partiellement saturés comprennent les groupes dérivés d'un dihydrothiophène, d'un dihydropyrane, d'un dihydrofurane ou d'un dihydrothiazole.

7. Composé conforme à l'une des revendications précédentes, dans lequel les substituants peuvent être choisis parmi les substituants hydroxy, oxo, halogéno, sulfanyle, nitro, amino, imino, cyano et formyle, les groupes, en option porteurs d'un ou de substituant(s), de type alkyle, halogéno-alkyle, hydroxy-alkyle, amino-alkyle, thio-alkyle, alcoxy, halogéno-alcoxy, alcoxy-alkyle, acyle et amino porteur d'un ou de deux substituant(s), et les groupes de type acide carboxylique et leurs dérivés tels que les groupes de type amide ou ester.

8. Composé conforme à l'une des revendications précédentes, choisi de préférence parmi les suivants :
S-cyclopentyl-S-phényl-N-{4-[2-éthyl-4-(3-méthyl-phényl)-1,3-thiazol-5-yl]-pyridine-2-yl}-sulfoximine ;
S,S-dicyclohexyl-N-{4-[2-éthyl-4-(m-tolyl)-thiazol-5-yl]-pyridine-2-yl}-sulfoximine ;
S-méthyl-S-(4-méthoxy-phényl)-N-{4-[2-éthyl-4-(m-tolyl)-thiazol-5-yl]-pyridine-2-yl}-sulfoximine ;
S-méthyl-S-phényl-N-{4-[2-éthyl-4-(3-méthyl-phényl)-1,3-thiazol-5-yl]-pyridine-2-yl}-sulfoximine ;
(+)-S-méthyl-S-phényl-N-{4-[2-éthyl-4-(3-méthyl-phényl)-1,3-thiazol-5-yl]-pyridine-2-yl}-sulfoximine ;
(-)-S-méthyl-S-phényl-N-{4-[2-éthyl-4-(3-méthyl-phényl)-1,3-thiazol-5-yl]-pyridine-2-yl}-sulfoximine ;
S-méthyl-S-(4-fluoro-phényl)-N-{4-[2-éthyl-4-(3-méthyl-phényl)-1,3-thiazol-5-yl]-pyridine-2-yl}-sulfoximine ;
S-isobutyl-S-phényl-N-{4-[2-éthyl-4-(m-tolyl)-thiazol-5-yl]-pyridine-2-yl}-sulfoximine ;
S-(3-fluoro-phényl)-S-méthyl-N-{4-[2-éthyl-4-(m-tolyl)-thiazol-5-yl]-pyridine-2-yl}-sulfoximine ;
S-(3-méthyl-phényl)-S-méthyl-N-{4-[2-éthyl-4-(m-tolyl)-thiazol-5-yl]-pyridine-2-yl}-sulfoximine ;
S,S-diphényl-N-{4-[2-éthyl-4-(m-tolyl)-thiazol-5-yl]-pyridine-2-yl}-sulfoximine ;
S-(2-méthyl-phényl)-S-méthyl-N-{4-[2-éthyl-4-(m-tolyl)-thiazol-5-yl]-pyridine-2-yl}-sulfoximine ;
S-isopropyl-S-phényl-N-{4-[2-éthyl-4-(3-méthyl-phényl)-1,3-thiazol-5-yl]-pyridine-2-yl}-sulfoximine ;
S-éthyl-S-phényl-N-{4-[2-éthyl-4-(3-méthyl-phényl)-l,3-thiazol-5-yl]-pyridine-2-yl}-sulfoximine ;
S-méthyl-S-(4-méthyl-phényl)-N-{4-[2-éthyl-4-(3-méthyl-phényl)-1,3-thiazol-5-yl]-pyridine-2-yl}-sulfoximine ;
di-n-butyl-N-{4-[2-éthyl-4-(3-méthyl-phényl)-1,3-thiazol-5-yl]-pyridine-2-yl}-sulfoximine ;
S-méthyl-S-(3-chloro-4-fluoro-phényl)-N-{4-[2-éthyl-4-(3-méthyl-phényl)-1,3-thiazol-5-yl]-pyridine-2-yl}-sulfoximine ;
1-imino-N-{4-[2-éthyl-4-(3-méthyl-phényl)-1,3-thiazol-5-yl]-pyridine-2-yl}-tétrahydro-2H-thiopyrane-1-oxyde ;
1-imino-N-{4-[2-éthyl-4-(3-méthyl-phényl)-1,3-thiazol-5-yl]-pyridine-2-yl}-tétrahydro-thiophène-1-oxyde ;
(-)-S-méthyl-S-phényl-N-[4-(2-éthyl-4-phényl-1,3-thiazol-5-yl)-pyridine-2-yl]-sulfoximine ;
(+)-S-méthyl-S-phényl-N-[4-(2-éthyl-4-phényl-1,3-thiazol-5-yl)-pyridine-2-yl]-sulfoximine ;
S-isopropyl-S-phényl-N-[4-(2-éthyl-4-phényl-1,3-thiazol-5-yl)-pyridine-2-yl]-sulfoximine ;
S-(4-méthoxy-phényl)-S-méthyl-N-[4-(2-éthyl-4-phényl-1,3-thiazol-5-yl)-pyridine-2-yl]-sulfoximine ;
S-méthyl-S-(3-méthyl-phényl)-N-[4-(2-éthyl-4-phényl-thiazol-5-yl)-pyridine]-sulfoximine ;
S-méthyl-S-(3-fluoro-phényl)-N-[4-(2-éthyl-4-phényl-thiazol-5-yl)-pyridine]-sulfoximine ;
4-(2-éthyl-4-phényl-thiazol-5-yl)-2-(dicyclohexyl-sulfoximine)-pyridine ;
S-méthyl-S-(4-fluoro-phényl)-N-[4-(2-éthyl-4-phényl-thiazol-5-yl)-pyridine]-sulfoximine ;
S-cyclopentyl-S-phényl-N-[4-(2-éthyl-4-phényl-thiazol-5-yl)-pyridine]-sulfoximine ;
(-)-S-cyclopentyl-S-phényl-N-[4-(2-éthyl-4-phényl-1,3-thiazol-5-yl)-pyridine-2-yl]-sulfoximine ;
(+)-S-cyclopentyl-S-phényl-N-[4-(2-éthyl-4-phényl-1,3-thiazol-5-yl)-pyridine-2-yl]-sulfoximine ;
S-méthyl-S-phényl-N-[4-(2-éthyl-4-phényl-1,3-thiazol-5-yl)-pyridine-2-yl]-sulfoximine ;
S,S-diphényl-N-[4-(2-éthyl-4-phényl-thiazol-5-yl)-pyridine-2-yl]-sulfoximine ;
(-)-S-méthyl-S-phényl-N-{4-[2-éthyl-4-(4-fluoro-phényl)-thiazol-5-yl]-pyridine-2-yl}-sulfoximine ;
S-(4-méthoxy-phényl)-S-méthyl-N-{4-[2-éthyl-4-(4-fluoro-phényl)-thiazol-5-yl]-pyridine-2-yl}-sulfoximine ;
S-isopropyl-S-phényl-N-{4-[2-éthyl-4-(4-fluoro-phényl)-thiazol-5-yl]-pyridine-2-yl}-sulfoximine ;
(-)-isopropyl-S-phényl-N-{4-[2-éthyl-4-(4-fluoro-phényl)-thiazol-5-yl]-pyridine-2-yl}-sulfoximine ;
(+)-S-isopropyl-S-phényl-N-{4-[2-éthyl-4-(4-fluoro-phényl)-thiazol-5-yl]-pyridine-2-yl}-sulfoximine ;
S-éthyl-S-phényl-N-{4-[2-éthyl-4-(4-fluoro-phényl)-thiazol-5-yl]-pyridine-2-yl}-sulfoximine ;
S-méthyl-S-phényl-N-{4-[2-éthyl-4-(4-fluoro-phényl)-1,3-thiazol-5-yl]-pyridine-2-yl}-sulfoximine ;
(+)-S-méthyl-S-phényl-N-{4-[2-éthyl-4-(4-fluoro-phényl)-1,3-thiazol-5-yl]-pyridine-2-yl}-sulfoximine ;
S-méthyl-S-(4-fluoro-phényl)-N-{4-[2-éthyl-4-(4-fluoro-phényl)-1,3-thiazol-5-yl]-pyridine-2-yl}-sulfoximine ;
S-cyclopentyl-S-phényl-N-{4-[2-éthyl-4-(4-fluoro-phényl)-1,3-thiazol-5-yl]-pyridine-2-yl}-sulfoximine ;
(-)-S-méthyl-S-(4-fluoro-phényl)-N-{4-[2-éthyl-4-(4-fluoro-phényl)-1,3-thiazol-5-yl]-pyridine-2-yl}-sulfoximine ;
(+)-S-méthyl-S-(4-fluoro-phényl)-N-{4-[2-éthyl-4-(4-fluoro-phényl)-1,3-thiazol-5-yl]-pyridine-2-yl}-sulfoximine ;
S,S-diphényl-N-{4-[2-éthyl-4-(4-fluoro-phényl)-thiazol-5-yl]-pyridine-2-yl}-sulfoximine ;
S-méthyl-S-(3-méthyl-phényl)-N-[4-(2-éthyl-4-fluorophényl-thiazol-5-yl)-pyridine]-sulfoximine ;
S-méthyl-S-(3-fluoro-phényl)-N-{4-[2-éthyl-4-(4-fluoro-phényl)-thiazol-5-yl]-pyridine-2-yl}-sulfoximine ;
S-cyclohexyl-S-phényl-N-{4-[2-éthyl-4-(4-fluoro-phényl)-thiazol-5-yl]-pyridine}-sulfoximine ;
S-méthyl-S-phényl-N-{4-[2-éthyl-4-(m-carboxy-phényl)-thiazol-5-yl]-pyridine-2-yl}-sulfoximine ;
S-méthyl-S-phényl-N-{4-[2-éthyl-4-(3-fluoro-phényl)-1,3-thiazol-5-yl]-pyridine-2-yl}-sulfoximine ;
S-isopropyl-S-phényl-N-{4-[2-éthyl-4-(3-fluoro-phényl)-1,3-thiazol-5-yl]-pyridine-2-yl}-sulfoximine ;
S-cyclopentyl-S-phényl-N-{4-[2-éthyl-4-(3-fluoro-phényl)-1,3-thiazol-5-yl]-pyridine-2-yl}-sulfoximine ;
S-méthyl-S-phényl-N-{4-[2-éthyl-4-(napht-1-yl)-thiazol-5-yl]-pyridine-2-yl}-sulfoximine ;
S-méthyl-S-phényl-N-{4-[2-éthyl-4-(3-trifluorométhyl-phényl)-thiazol-5-yl]-pyridine-2-yl}-sulfoximine ;
S-méthyl-S-phényl-N-{4-[2-éthyl-4-(4-méthoxy-phényl)-thiazol-5-yl]-pyridine-2-yl}-sulfoximine ;
S-méthyl-S-phényl-N-{4-[2-(4-méthylsulfanyl-phényl)-4-(m-tolyl)-thiazol-5-yl]-pyridine-2-yl}-sulfoximine ;
S-méthyl-S-phényl-N-{4-[2-(4-méthylsulfinyl-phényl)-4-(m-tolyl)-thiazol-5-yl]-pyridine-2-yl}-sulfoximine ;
S-méthyl-S-phényl-N-{4-[2-éthyl-4-(3,4-difluoro-phényl)-thiazol-5-yl]-pyridine-2-yl}-sulfoximine ;
S-méthyl-(4-fluoro-phényl)-N-{4-[2-éthyl-4-(3,4-difluoro-phényl)-thiazol-5-yl]-pyridine-2-yl}-sulfoximine ;
S-isopropyl-S-phényl-N-{4-[2-éthyl-4-(3,4-difluoro-phényl)-thiazol-5-yl]-pyridine-2-yl}-sulfoximine ;
S-isopropyl-S-phényl-N-{4-[2-éthyl-4-(3,5-difluoro-phényl)-thiazol-5-yl]-pyridine-2-yl}-sulfoximine ;
S-méthyl-S-phényl-N-{4-[2-éthyl-4-(3,5-difluoro-phényl)-thiazol-5-yl]-pyridine-2-yl}-sulfoximine ;
S,S-diphényl-N-{4-[2-éthyl-4-(3,5-difluoro-phényl)-thiazol-5-yl]-pyridine-2-yl}-sulfoximine ;
S-méthyl-S-phényl-N-{4-[2-éthyl-4-(p-tolyl)-thiazol-5-yl]-pyridine-2-yl}-sulfoximine ;
S-cyclopentyl-S-phényl-N-{4-[2-(2,6-difluoro-phényl)-4-(3-fluoro-phényl)-1,3-thiazol-5-yl]-pyridine-2-yl}-sulfoximine ;
S-isopropyl-S-phényl-N-{4-[2-(2,6-difluoro-phényl)-4-(3-fluoro-phényl)-1,3-thiazol-5-yl]-pyridine-2-yl}-sulfoximine ;
S-cyclohexyl-S-phényl-N-{4-[2-(2,6-difluoro-phényl)-4-(3-fluoro-phényl)-1,3-thiazol-5-yl]-pyridine-2-yl}-sulfoximine ;
S-cyclohexyl-S-phényl-N-{4-[2-(4-fluoro-phényl)-4-(3-fluoro-phényl)-1,3-thiazol-5-yl]-pyridine-2-yl}-sulfoximine ;
S-isopropyl-S-phényl-N-{4-[2-(4-fluoro-phényl)-4-(3-fluoro-phényl)-1,3-thiazol-5-yl]-pyridine-2-yl}-sulfoximine ;
sel méthane-sulfonate de (+)-S-isopropyl-S-phényl-N-{4-[2-éthyl-4-(4-fluoro-phényl)-1,3-thiazol-5-yl]-pyridine-2-yl}-sulfoximine ;
N-oxyde du sel méthane-sulfonate de (+)-S-isopropyl-S-phényl-N-{4-[2-éthyl-4-(4-fluoro-phényl)-l,3-thiazol-5-yl]-pyridine-2-yl}-sulfoximine ;
S-cyclopentyl-S-phényl-N-{[4-(3-fluoro-phényl)-1,3-thiazol-5-yl]-pyridine-2-yl}-sulfoximine ;
S-méthyl-S-phényl-N-{[4-(3-fluoro-phényl)-1,3-thiazol-5-yl]-pyridine-2-yl}-sulfoximine ;
S-isopropyl-S-phényl-N-{[4-(3-fluoro-phényl)-1,3-thiazol-5-yl]-pyridine-2-yl}-sulfoximine ;
S-méthyl-S-phényl-N-{[4-(4-fluoro-phényl)-1,3-thiazol-5-yl]-pyridine-2-yl}-sulfoximine ;
S-isopropyl-S-phényl-N-{[4-(4-fluoro-phényl)-1,3-thiazol-5-yl]-pyridine-2-yl}-sulfoximine ;
S-cyclopentyl-S-phényl-N-{[4-(4-fluoro-phényl)-1,3-thiazol-5-yl]-pyridine-2-yl}-sulfoximine ;
S-méthyl-S-phényl-N-[(4-phényl-1,3-thiazol-5-yl)-pyridine-2-yl]-sulfoximine ;
S-isopropyl-S-phényl-N-[(4-phényl-1,3-thiazol-5-yl)-pyridine-2-yl]-sulfoximine ;
S-cyclopentyl-S-phényl-N-[(4-phényl-thiazol-5-yl)-pyridine-2-yl]-sulfoximine.

9. Composition pharmaceutique qui comprend un composé de formule (I), conforme à l'une des revendications précédentes, ainsi qu'un excipient, diluant ou véhicule pharmacologiquement admissible.

10. Procédé de préparation d'un composé de formule (I), conforme à l'une des revendications précédentes, comprenant l'étape consistant à faire réagir un composé de formule (II), dans laquelle X représente un groupe partant approprié, avec une sulfoximine de formule (III), à l'aide d'un ou de catalyseur(s) de couplage approprié(s), choisi(s) parmi l'acétate de palladium et les sels de cuivre en présence d'un ou de ligand(s) approprié(s) choisi(s) parmi la N,N'-diméthyl-éthylène-diamine (DMEDA), et en présence d'une ou de base(s) inorganique(s) appropriée(s) choisie(s) parmi les carbonate de césium, acétate de césium, carbonate de potassium, phosphate de potassium, hydroxyde de potassium, hydroxyde de sodium, carbonate de sodium, hydroxyde de lithium, hydrure de sodium et hydrure de potassium, ainsi que leurs mélanges, de manière à obtenir un composé de formule (I), étant entendu que chacun des termes a la signification indiquée dans la description.

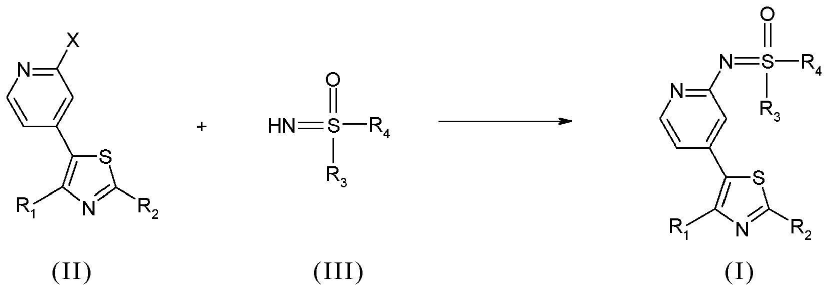

11. Composé conforme à l'une des revendications 1 à 8, ou composition conforme à la revendication 9, pour utilisation dans un traitement thérapeutique du corps d'un humain ou d'un animal.

12. Composé conforme à l'une des revendications 1 à 8, ou composition conforme à la revendication 9, pour utilisation dans un procédé de traitement de la polyarthrite rhumatoïde.

13. Utilisation d'un composé conforme à l'une des revendications 1 à 8, ou d'une composition conforme à la revendication 9, dans la fabrication d'un médicament destiné à être utilisé dans le traitement de la polyarthrite rhumatoïde.
